# EUROPEAN PATENT APPLICATION

(11) **EP 4 339 197 A1**
(43) Date of publication of application: **20.03.2024**
(21) Application number: 22828827.0
(22) Date of filing: 24.06.2022
(51) Int. Cl.: C07D 471/04, A61K 31/437, A61K 31/506, A61K 31/444, A61K 31/4725, A61K 31/501, A61K 31/4545, A61P 35/00, A61P 37/00

(54) **NOVEL UREA DERIVATIVE COMPOUND AS RON INHIBITOR**

(30) Priority: 24.06.2021 KR 20210082700
(71) Applicant: LG Chem, Ltd., Seoul 07336 (KR)
(72) Inventor: HAM, Young Jin, Daejeon 34122 (KR); CHO, Joong Heui, Daejeon 34122 (KR); YOON, Hong Bin, Daejeon 34122 (KR); KIM, Jung Joon, Daejeon 34122 (KR); KWAK, Young Shin, Daejeon 34122 (KR); KIM, Gyeong Hwan, Daejeon 34122 (KR); LEE, Ju Hyun, Daejeon 34122 (KR)
(74) Representative: Hoffmann Eitle
(86) International application number: PCT/KR2022/009043
(87) International publication number: WO 2022/270980

(57) **Abstract**

The present invention relates to a novel compound as a protein kinase inhibitor, particularly, a RON inhibitor.

## Description

### TECHNICAL FIELD

### Cross-reference to Related Applications

This application claims the benefit of Korean Patent Application No. 10-2021-0082700, filed on June 24, 2021, in the Korean Intellectual Property Office, the disclosure of which is incorporated herein in its entirety by reference.

### Technical Field

The present invention relates to a novel compound as a protein kinase inhibitor. Specifically, the present invention relates to a novel compound as a RON inhibitor.

### BACKGROUND ART

At least 400 protein kinases that catalyze a phosphoryl transition reaction from adenosine triphosphate (ATP) to a protein substrate are known. In a target protein to which the phosphoryl group is transferred, a specific amino acid is tyrosine, serine, or threonine, so that protein kinases are commonly referred to as protein tyrosine kinases (PTKs) or serine/threonine kinases (STKs).

The protein kinases constitute a large family of structurally related groups that are responsible for the control of a wide variety of signaling pathways within the cell. The signaling pathways include many other signaling pathways by the transfer of the phosphoryl group to the target protein. These phosphorylation events act as molecular on/off switches that can regulate the biological function of the target protein or protein complex. The appropriate protein kinase functions in signaling pathways are to activate or deactivate metabolic enzymes, regulatory proteins, receptors, cytoskeletal proteins, ion channels and pumps, transcription factors, and the like. Uncontrolled signaling due to defective control of protein phosphorylation is implicated in a number of diseases, including inflammation, cancer, allergy/asthma, diseases of the immune system, diseases of the central nervous system, angiogenesis, and the like.

Almost all kinases include a similar 250-300 amino acid catalytic domain. The kinases may be categorized by the substrates they phosphorylate, and sequence motifs have been identified that generally correspond to each of these kinase families.

Meanwhile, a receptor originated from nantes (RON), which is one of tyrosine protein kinase receptors, is also referred to as a macrophage stimulating 1 receptor (MST1R), and has been reported to promote the invasion and metastasis of cancer cells. Overexpression of RON is also known to appear in a variety of tumor types.

Activation of tyrosine protein kinases such as the RON in tumor cells increases the proliferation, invasion, and metastasis of tumor cells, and increases the resistance of tumor cells to apoptosis and cytotoxic therapy. Therefore, a selective small molecule kinase modulator targeting a tyrosine protein kinase such as the RON is expected to have therapeutic potential for the treatment of cancers in which activation of RON receptors and the like plays a critical role in the development and progression of primary tumors and secondary metastases. Accordingly, continuous research is being conducted on various inhibitors for selectively inhibiting kinase activity in RON, one of the tyrosine proteins.

### DISCLOSURE OF THE INVENTION

### TECHNICAL PROBLEM

An aspect of the present invention provides a novel compound with protein kinase inhibitory activity.

Another aspect of the present invention provides a compound useful for the prevention or treatment of cancers.

Yet another aspect of the present invention provides a compound useful for the prevention or treatment of immune-related diseases.

Still another aspect of the present invention provides a compound useful as a RON inhibitor.

### TECHNICAL SOLUTION

In order to achieve the above objective,
according to an aspect of the present invention, there is provided a pyridine derivative compound of Formula 1 below or a pharmaceutically acceptable salt thereof.

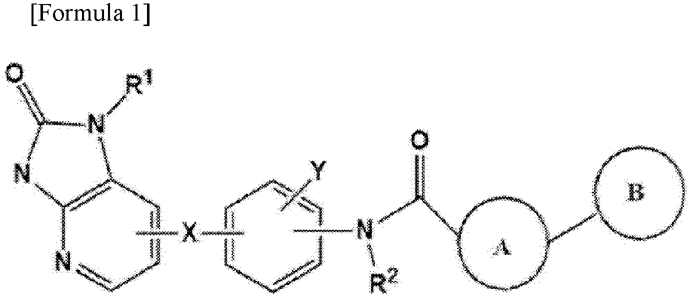

In Formula 1,
X above is O,
Y above is hydrogen, halogen, C₁-C₆ alkyl, or C₃-C₆ cycloalkyl,
R¹ above is hydrogen, C₁-C₆ alkyl, C₃-C₁₂ cycloalkyl, or C₂-C₁₁ heterocycloalkyl,
R² above is hydrogen or C₁-C₆ alkyl,
A above is -Ar or -(CH₂)n-Ar, wherein Ar is substituted or unsubstituted C₆-C₁₀ aryl, or substituted or unsubstituted 5-membered heteroaryl including 1 to 4 heterocyclic atoms selected from the group consisting of nitrogen, sulfur, and oxygen, wherein a substituent is one or more selected from halogen, amine, C₁-C₆ alkyl, C₃-C₁₂ cycloalkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR³R⁴, and n above is an integer of 1 to 3,
R³ and R⁴ above are each independently hydrogen or C₁-C₆ alkyl,
B above is hydrogen, C₁-C₆ alkyl, substituted C₁-C₆ alkyl, C₃-C₁₂ cycloalkyl, substituted C₃-C₁₂ cycloalkyl, C₃-C₁₁ heterocycloalkyl, substituted C₃-C₁₁ heterocycloalkyl, C₆-C₂₀ aryl, substituted C₆-C₂₀ aryl, wherein a substituent is halogen, C₁-C₆ alkyl, C₃-C₇ cycloalkyl, halogen-substituted C₁-C₆ alkyl, C₁-C₆ alkoxy, C₃-C₁₁ heterocycloalkenyl, or C₆-C₁₀ aryl,
wherein the halogen is selected from the group consisting of F, Cl, Br, and I.

According to another aspect of the present invention, there is provided a pharmaceutical composition including the compound of Formula 1 above or the pharmaceutically acceptable salt thereof, and a pharmaceutically acceptable carrier.

### ADVANTAGEOUS EFFECTS

According to the present invention, a novel compound or a pharmaceutically acceptable salt thereof which may be usefully used in the treatment of various immunemediated diseases, including anticancer agents, by inhibiting a protein kinase, the protein kinase activity of a RON receptor in particular, but the present invention is not limited thereto.

### MODE FOR CARRYING OUT THE INVENTION

According to one aspect, the present invention provides a novel urea derivative compound of Formula 1 defined above or a pharmaceutically acceptable salt thereof.

In the present invention, the compound include, but is not limited to, a compound of Formula 1, a stereoisomer such as an enantiomer and a diastereomer, a solvate, and a prodrug thereof.

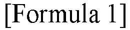

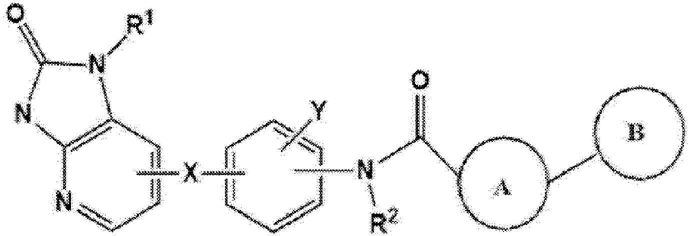

In Formula 1,
X above is O,
Y above is hydrogen, halogen, C₁-C₆ alkyl, or C₃-C₆ cycloalkyl,
R¹ above is hydrogen, C₁-C₆ alkyl, C₃-C₁₂ cycloalkyl, or C₂-C₁₁ heterocycloalkyl,
R² above is hydrogen or C₁-C₆ alkyl,
A above is -Ar or -(CH₂)n-Ar, wherein Ar is substituted or unsubstituted C₆-C₁₀ aryl, or substituted or unsubstituted 5-membered heteroaryl including 1 to 4 heterocyclic atoms selected from the group consisting of nitrogen, sulfur, and oxygen, wherein a substituent is one or more selected from halogen, amine, C₁-C₆ alkyl, C₃-C₁₂ cycloalkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR³R⁴, and n above is an integer of 1 to 3,
R³ and R⁴ above are each independently hydrogen or C₁-C₆ alkyl,
B above is hydrogen, C₁-C₆ alkyl, substituted C₁-C₆ alkyl, C₃-C₁₂ cycloalkyl, substituted C₃-C₁₂ cycloalkyl, C₃-C₁₁ heterocycloalkyl, substituted C₃-C₁₁ heterocycloalkyl, C₆-C₂₀ aryl, substituted C₆-C₂₀ aryl, wherein a substituent is halogen, C₁-C₆ alkyl, C₃-C₇ cycloalkyl, halogen-substituted C₁-C₆ alkyl, C₁-C₆ alkoxy, C₃-C₁₁ heterocycloalkenyl, or C₆-C₁₀ aryl,
wherein the halogen is selected from the group consisting of F, Cl, Br, and I.

In the present specification, the term "substitution" means that a hydrogen atom bonded to a carbon atom in a structure is changed to another substituent, and the position at which the substitution takes place is not limited as long as it is a position at which the hydrogen atom is substituted, that is, a position at which a substituent may be substituted, and when substituted at two or more positions, two or more substituents may be the same as or different from each other.

In the present specification, unless otherwise defined, a "substituent" may be one or more selected from the group consisting of deuterium, halogen, hydroxy, C₁-C₁₀ alkyl, C₃-C₁₂ cycloalkyl, C₁-C₁₀ alkoxy, C₅-C₁₂ aryloxy, C₁-C₁₀ alkylthioxy, C₅-C₁₂ arylthioxy, C₁-C₁₀ alkylsulfoxy, C₅-C₁₂ arylsulfoxy, C₁-C₁₀ haloalkyl, C₂-C₂₀ alkenyl, C₀-C₁₀ amine, nitrile, nitro, imide, amide, oxo, carbonyl, carboxylic acid, carbamoyl, ester, C₅-C₁₂ aryl, and C₅-C₁₂ heteroaryl.

In the present specification, the "alkyl" may be a straight chain or branched chain, and preferably has 1 to 20 carbon atoms unless otherwise defined. Examples thereof may include methyl, ethyl, propyl, n-propyl, isopropyl, butyl, n-butyl, isobutyl, tert-butyl, secbutyl, 1-methylbutyl, ethylbutyl, pentyl, n-pentyl, isopentyl, neopentyl, tert-pentyl, hexyl, n-hexyl, methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tertoctyl, 1-methylheptyl, 2-ethylhexyl, 2-propylpentyl, n-nonyl, 2,2-dimethylheptyl, 1-ethylpropyl, dimethylpropyl, isohexyl, 2-methylpentyl, 4-methylhexyl, 5-methylhexyl, and the like, but are not limited thereto.

In the present specification, the "cycloalkyl" may mean a cyclic saturated hydrocarbon, and preferably have 3 to 20 carbon atoms unless otherwise defined. Examples thereof may include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2, 3-dimethylcyclohexyl, 3,4,5-trimethylcyclohexyl, 4-tert-butylcyclohexyl, cycloheptyl, cyclooctyl, and the like, but are not limited thereto.

In the present specification, the "alkoxy" group may be a straight, branched, or cyclic, and preferably has carbon atoms of 1 to 20 unless otherwise defined. Examples thereof may include methoxy, ethoxy, n-propoxy, isopropoxy, n-butoxy, isobutoxy, tertbutoxy, sec-butoxy, n-pentyloxy, neopentyloxy, isopentyl oxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the "alkenyl" group may be a straight chain or branched chain, and preferably has 2 to 20 carbon atoms unless otherwise defined. Examples thereof may include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl- 1-yl)vinyl-1-yl, 2,2-bis(diphenyl-1-yl)vinyl-1-yl, a stilbenyl group, a styrenyl group, and the like, but are not limited thereto.

In the present specification, the "aryl" may be monoaryl, biaryl, or polycyclic aryl of three or more rings, and when two or more cyclic structures are included, each ring may be fused or included in a spiro form, and preferably has 5 to 12 carbon atoms unless otherwise defined. Examples thereof may include a phenyl group, a biphenyl group, a terphenyl group, a naphthyl group, an anthracenyl group, a phenanthryl group, a pyrenyl group, a perylenyl group, and the like, but are not limited thereto.

In the present specification, the "heterocyclic atom" means a non-carbon atom included in a ring. Unless otherwise defined, the heterocyclic atom may include one or more atoms selected from oxygen, nitrogen, selenium, and sulfur.

In the present specification, the "heterocycloalkyl" may mean a cyclic saturated hydrocarbon including a heterocyclic atom, and preferably has 2 to 20 carbon atoms unless otherwise defined.

In the present specification, the "heteroaryl" may mean an aromatic hydrocarbon including a heterocyclic atom. The heteroaryl may be monocyclic or polycyclic, and when including two or more cyclic structures, each ring may be fused or included in a spiro form, and preferably has 5 to 12 carbon atoms unless otherwise defined. Examples thereof may include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group , a pyridazinyl group, a pyrazinyl group, a quinolinyl group, a quinazolinyl group, a quinoxalinyl group, a phthalazinyl group, a pyrido pyrimidyl group, a pyrido pyrazinyl group, a pyrazino pyrazinyl group, an isoquinolinyl group, an indolyl group, a carbazolyl group, a benzoxazolyl group, a benzimidazolyl group, a benzothiazolyl group, and the like, but are not limited thereto.

According to an embodiment, the present invention provides a novel urea derivative compound of Formula 2 below or a pharmaceutically acceptable salt thereof.

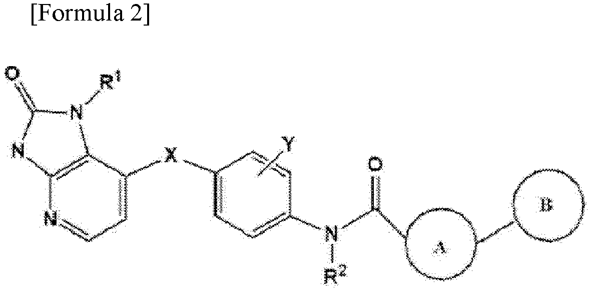

The substituent is the same as defined in Formula 1.

According to an embodiment, in Formula 1 above, A above may be -Ar or - (CH₂)n-Ar, wherein Ar above may be substituted or unsubstituted C₆-C₁₀ aryl or substituted or unsubstituted 5-membered heteroaryl. At this time, in regard to the structure A, the term 'aryl' does not mean only the structure in which A is a monovalent functional group, that is, a structure in which B is hydrogen in Formula 1 above, and when B above has a structure other than hydrogen, Ar above may be substituted or unsubstituted C₆-C₁₀ arylene or substituted or unsubstituted 5-membered heteroarylene.

According to an embodiment, in Formula 1 above, A above may be a substituted or unsubstituted 5-membered heteroaryl, and specifically, may include at least two heterocyclic atoms. The heterocyclic atoms included at this time may be the same or different from each other, but are not limited thereto.

According to another embodiment, in Formula 1 above, Ar above may include at least one nitrogen atom as a heterocyclic atom, and may further include at least one heterocyclic atom selected from nitrogen and sulfur.

According to another embodiment, Ar above may include one or two heteroaryl groups selected from the group consisting of pyrazole, imidazole, and thiazole, wherein the heteroaryl group may be substituted or unsubstituted, and a substituent which the substituted heteroaryl group has may be halogen, amine, C₁-C₆ alkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR³R⁴. At this time, R³ and R⁴ above are as defined above.

According to another embodiment, in Formula 1 above, A above may be a substituted or unsubstituted C₆-C₁₀ aryl, and specifically, may be a substituted or unsubstituted phenyl. At this time, a substituent which the substituted phenyl has may be one or more selected from halogen and halogen-substituted C₁-C₆ alkyl. More specifically, a substituent which the substituted phenyl has may be one or more selected from fluoro, bromo, and trifluoromethyl.

According to another embodiment, in Formula 1 above, A may be -(CH₂)n-Ar, wherein n may be an integer of 1 to 3, and for example, n may be 1.

According to an embodiment, in Formula 1 above, B above may be hydrogen, C₁-C₆ alkyl, substituted C₁-C₆ alkyl, C₃-C₇ cycloalkyl, phenyl, substituted phenyl, benzyl, or naphthalene tetrahydropyran, piperidine or substituted piperidine, wherein a substituent may be halogen, C₁-C₃ alkyl, halogenated C₁-C₃ alkyl, C₃-C₅ cycloalkyl, or C₁-C₃ alkoxy.

According to an embodiment, in Formula 1 above, Y above is fluoro.

According to an embodiment, in Formula 1 above, R¹ above is C₁-C₆ alkyl, C₃-C₆ cycloalkyl, or C₄-C₆ heterocycloalkyl.

According to another embodiment, in Formula 1 above, R¹ above is hydrogen, methyl, ethyl, isopropyl, cyclopentyl, or tetrahydrofuranyl.

According to an embodiment, in Formula 1 above, R² above is hydrogen.

According to an embodiment, the compound of Formula 1 above may be selected from the group consisting of
N-(3-fluoro-4-((2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide,
N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide,
N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-pyrazole-4-carboxamide,
5-ethyl-N-(3-fluoro-4-((2-oxo-2,3-dihydro-1H-imidazo[4,5-b]pyridine-7-yl)oxy)phenyl)-1-phenyl-1H-pyrazole-4-carboxamide,
5-ethyl-N-(3-fluoro-4-((1-isopropyl-2-oxo-2,3-dihydro-1H-imidazo[4,5-b]pyridine-7-yl)oxy)phenyl)-1-phenyl-1H-pyrazole-4-carboxamide,
N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-imidazole-4-carboxamide,
N-(3-fluoro-4-((2-oxo-2,3-dihydro-1H-imidazo[4,5-b]pyridine-7-yl)oxy)phenyl)-3,5-dimethyl-1-phenyl-1H-pyrazole-4-carboxamide,
N-(3-fluoro-4-((1-isopropyl-2-oxo-2,3-dihydro-1H-imidazo[4,5-b]pyridine-7-yl)oxy)phenyl)-3, 5-dimethyl-1-phenyl-1H-pyrazole-4-carboxamide,
N-(3-fluoro-4-((1-isopropyl-2-oxo-2,3-dihydro-1H-imidazo[4,5-b]pyridine-7-yl)oxy)phenyl)-5-methyl-1-phenyl-1H-pyrazole-4-carboxamide,
N-(3-fluoro-4-((1-isopropyl-2-oxo-2,3-dihydro-1H-imidazo[4,5-b]pyridine-7-yl)oxy)phenyl)-2-phenylthiazole-5-carboxamide,
N-(3-fluoro-4-((1-isopropyl-2-oxo-2,3-dihydro-1H-imidazo[4,5-b]pyridine-7-yl)oxy)phenyl)-5-phenylthiazole-2-carboxamide,
N-(3-fluoro-4-((1-isopropyl-2-oxo-2,3-dihydro-1H-imidazo[4,5-b]pyridine-7-yl)oxy)phenyl)-1-(p-tolyl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-(2-methoxyphenyl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1, 5-diphenyl-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-(tetrahydro-2H-pyran-3-yl)-5-(trifluoromethyl)-1H-pyrazole-4-carboxamide,
N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide,
N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide,
N-(4-((1-cyclopentyl-2-oxo-2,3-dihydro-1H-imidazo[4,5-b]pyridine-7-yl)oxy)-3-fluorophenyl)-5-ethyl-1-phenyl-1H-pyrazole-4-carboxamide,
N-(3-fluoro-4-((1-isopropyl-2-oxo-2,3-dihydro-1H-imidazo[4,5-b]pyridine-7-yl)oxy)phenyl)-2-(trifluoromethyl)benzamide,
4-bromo-N-(3-fluoro-4-((1-isopropyl-2-oxo-2,3-dihydro-1H-imidazo[4,5-b]pyri dine-7-yl)oxy)phenyl)-2-(trifluoromethyl)benzamide,
N-(3-fluoro-4-((1-isopropyl-2-oxo-2,3-dihydro-1H-imidazo[4,5-b]pyridine-7-yl)oxy)phenyl)-1-(naphthalene-1-yl)-5-(trifluoromethyl)-1H-pyrazole-4-carboxamide,
3-bromo-N-(3-fluoro-4-((1-isopropyl-2-oxo-2,3-dihydro-1H-imidazo[4,5-b]pyridine-7-yl)oxy)phenyl)-2-(trifluoromethyl)benzamide,
1-cyclohexyl-N-(3-fluoro-4-((1-isopropyl-2-oxo-2,3-dihydro-1H-imidazo[4,5-b]pyridine-7-yl) Oxy)phenyl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-(tetrahydro-2H-pyran-4-yl)-5-(trifluoromethyl)-1H-pyrazole-4-carboxamide,
1-(tert-butyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
1-cycloheptyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-2-(1-phenyl-5-(trifluoromethyl)-1H-pyrazol-4-yl)acetamide,
N-(3-fluoro-4-((1-isopropyl-2-oxo-2,3-dihydro-1H-imidazo[4,5-b]pyridine-7-yl)oxy)phenyl)-1-(1-methylpiperidine-3-yl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-(2fluorophenyl)-5-(trifluoromethyl)-1H-pyrazole-4-carboxamide,
1-(2-chlorophenyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
1-(2,6-difluorophenyl)-N-(3-fluoro-4-((1-isopropyl-2-oxo-2,3-dihydro-1H-imidazo[4,5- b]pyridine-7-yl)oxy)phenyl)-5-(trifluoromethyl)-1H-pyrazole-4-carboxamide,
N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((2-oxo-1-(tetrahydrofuran-3-yl)-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-(1-methylpiperidine-4-yl)-2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-phenyl-1H-pyrazole-3-carboxamide,
N-(3-fluoro-4-((1-isopropyl-2-oxo-2,3-dihydro-1H-imidazo[4,5-b]pyridine-7-yl)oxy)phenyl)-1-phenyl-1H-1,2,3-triazole-4-carboxamide,
N-(3-fluoro-4-((1-isopropyl-2-oxo-2,3-dihydro-1H-imidazo[4,5-b]pyridine-7-yl)oxy)phenyl)-5-methyl-1-phenyl-1H-1,2,3-triazole-4-carboxamide,
   and
N-(3-fluoro-4-((1-isopropyl-2-oxo-2,3-dihydro-1H-imidazo[4,5-b]pyridine-7-yl)oxy)phenyl)-1-(2-fluorophenyl)-1H-1,2,3-triazole-4-carboxamide,
and the structure thereof is as shown in Table 1 below.

**[Table 1]**

| | Structure | IUPAC name |
|---|---|---|
| Example 1 | 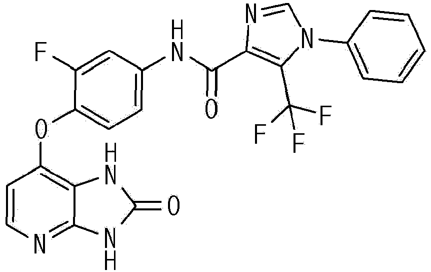 | N-(3-fluoro-4-((2-oxo-2,3-dihydro-1H-imidazo[4,5-b]pyridine-7-yl)oxy)phenyl)-1-phenyl-5-(trifluoromethyl)-1H-imidazole-4-carboxamide |
| Example 2 | 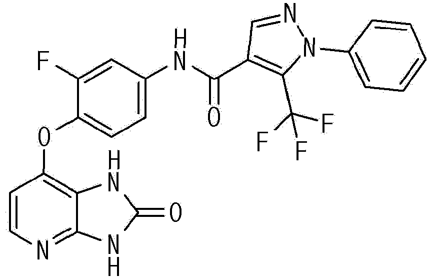 | N-(3-fluoro-4-((2-oxo-2,3-dihydro-1H-imidazo[4,5-b]pyridine-7-yl)oxy)phenyl)-1-phenyl-5-(trifluoromethyl)-1H-pyrazole-4-carboxamide |
| Example 3 | 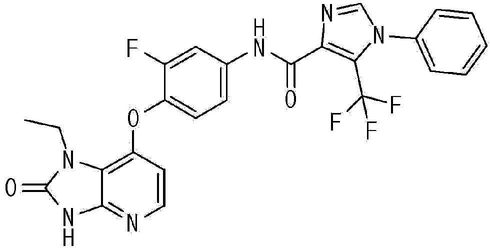 | N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide |
| Example 4 | 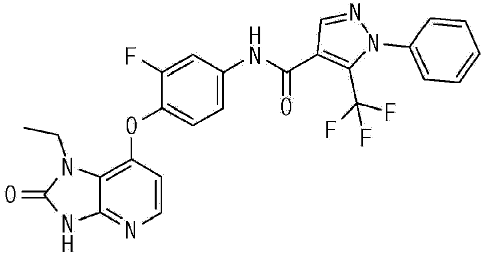 | N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide |
| Example 5 | 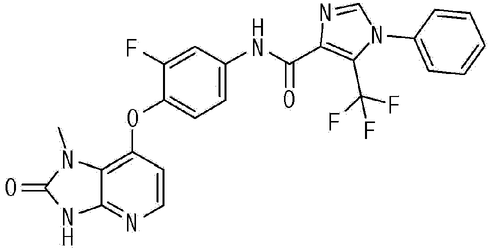 | N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-imidazole-4-carboxamide |
| Example 6 | 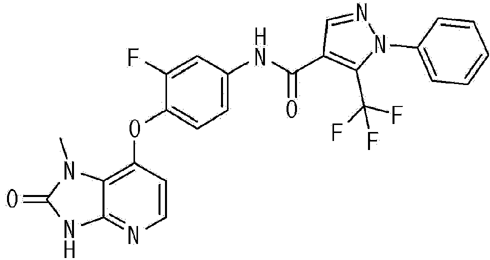 | N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-pyrazole-4-carboxamide |
| Example 7 | 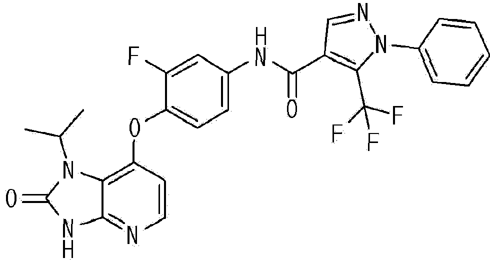 | N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-pyrazole-4-carboxamide |
| Example 8 | 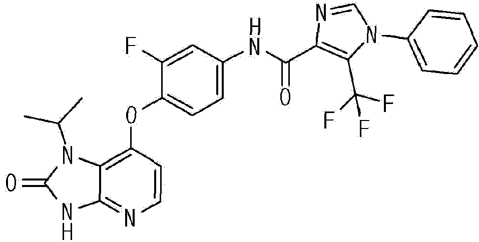 | N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-imidazole-4-carboxamide |
| Example 9 | 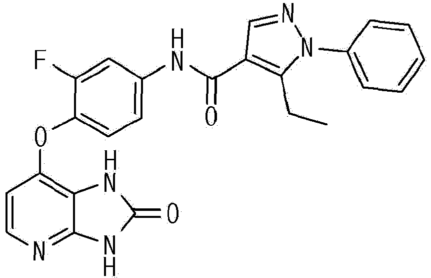 | 5-ethyl-N-(3-fluoro-4-((2-oxo-2,3-dihydro-1H-imidazo[4,5-b]pyridine-7-yl)oxy)phenyl)-1-phenyl-1H-pyrazole-4-carboxamide |
| Example 10 | 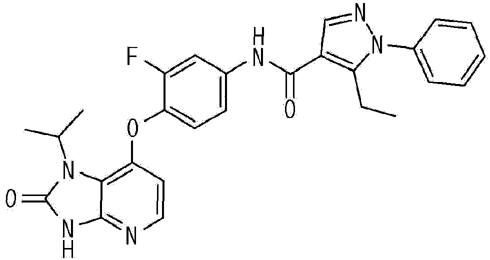 | 5-ethyl-N-(3-fluoro-4-((1-isopropyl-2-oxo-2,3-dihydro-1H-imidazo[4,5-b]pyridine-7-yl)oxy)phenyl)-1-phenyl-1H-pyrazole-4-carboxamide |
| Example 11 | 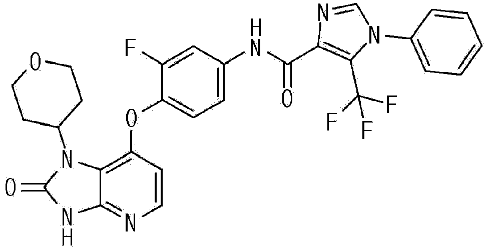 | N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-imidazole-4-carboxamide |
| Example 12 | 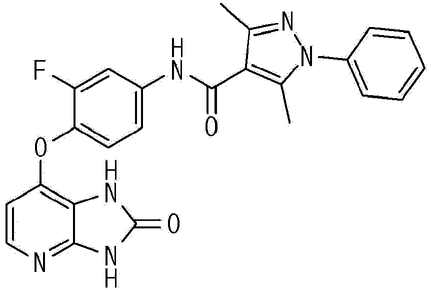 | N-(3-fluoro-4-((2-oxo-2,3-dihydro-1H-imidazo[4,5-b]pyridine-7-yl)oxy)phenyl)-3,5-dimethyl-1-phenyl-1H-pyrazole-4-carboxamide |
| Example 13 | 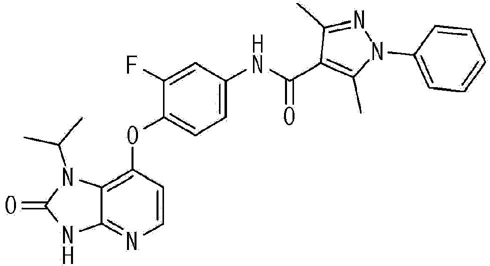 | N-(3-fluoro-4-((1-isopropyl-2-oxo-2,3-dihydro-1H-imidazo[4,5-b]pyridine-7-yl)oxy)phenyl)-3,5-dimethyl-1-phenyl-1H-pyrazole-4-carboxamide |
| Example 14 | 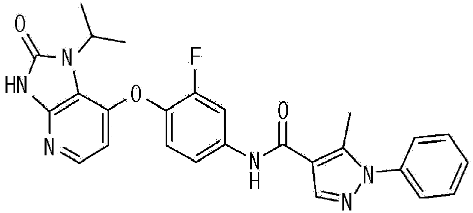 | N-(3-fluoro-4-((1-isopropyl-2-oxo-2,3-dihydro-1H-imidazo[4,5-b]pyridine-7-yl)oxy)phenyl)-5-methyl-1-phenyl-1H-pyrazole-4-carboxamide |
| Example 15 | 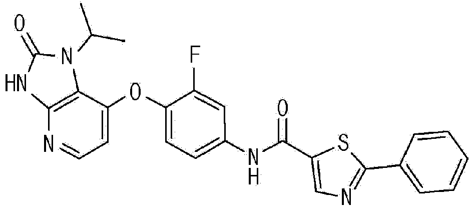 | N-(3-fluoro-4-((1-isopropyl-2-oxo-2,3-dihydro-1H-imidazo[4,5-b]pyridine-7-yl)oxy)phenyl)-2-phenylthiazole-5-carboxamide |
| Example 16 | 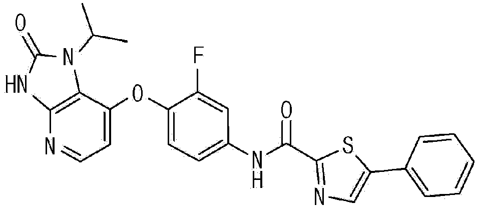 | N-(3-fluoro-4-((1-isopropyl-2-oxo-2,3-dihydro-1H-imidazo[4,5-b]pyridine-7-yl)oxy)phenyl)-5-phenylthiazole-2-carboxamide |
| Example 17 | 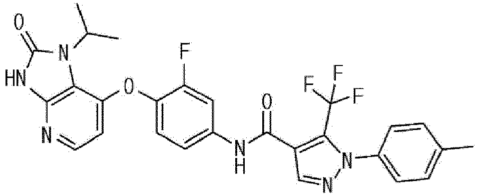 | N-(3-fluoro-4-((1-isopropyl-2-oxo-2,3-dihydro-1H-imidazo[4,5-b]pyridine-7-yl)oxy)phenyl)-1-(p-tolyl)-5-(trifluoromethyl)-1H-pyrazole-4-carboxamide |
| Example 18 | 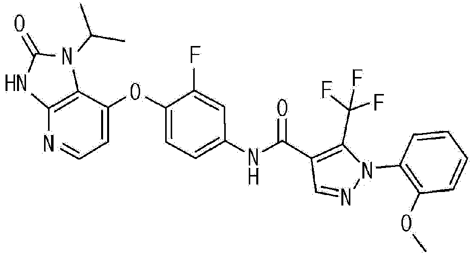 | N-(3-fluoro-4-((1-isopropyl-2-oxo-2,3-dihydro-1H-imidazo[4,5-b]pyridine-7-yl)oxy)phenyl)-1-(2-methoxyphenyl)-5-(trifluoromethyl)-1H-pyrazole-4-carboxamide |
| Example 19 | 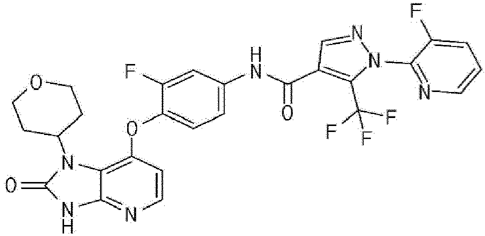 | N-(3-fluoro-4-((1-isopropyl-2-oxo-2,3-dihydro-1H-imidazo[4,5-b]pyridine-7-yl)oxy)phenyl)-1,5-diphenyl-1H-pyrazole-4-carboxamide |
| Example 20 | 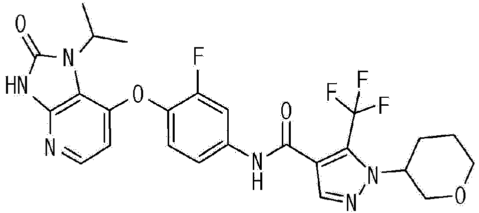 | N-(3-fluoro-4-((1-isopropyl-2-oxo-2,3-dihydro-1H-imidazo[4,5-b]pyridine-7-yl)oxy)phenyl)-1-(tetrahydro-2H-pyran-3-yl)-5-(trifluoromethyl)-1H-pyrazole-4-carboxamide |
| Example 21 | 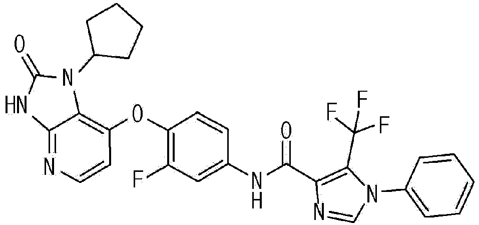 | N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide |
| Example 22 | 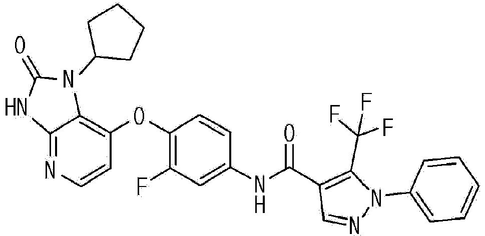 | N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide |
| Example 23 | 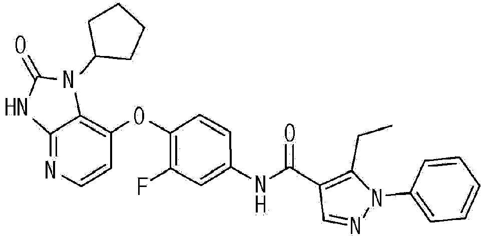 | N-(4-((1-cyclopentyl-2-oxo-2,3-dihydro-1H-imidazo[4,5-b]pyridine-7-yl)oxy)-3-fluorophenyl)-5-ethyl-1-phenyl-1H-pyrazole-4-carboxamide |
| Example 24 | 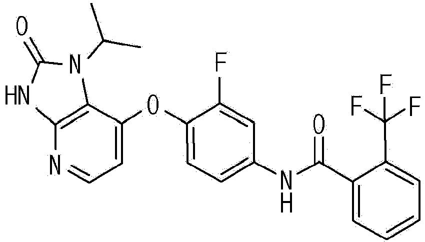 | N-(3-fluoro-4-((1-isopropyl-2-oxo-2,3-dihydro-1H-imidazo[4,5-b]pyridine-7-yl)oxy)phenyl)-2-(trifluoromethyl)benzamide |
| Example 25 | 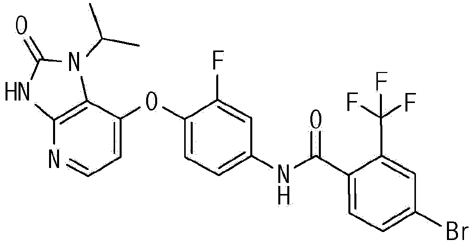 | 4-bromo-N-(3-fluoro-4-((1-isopropyl-2-oxo-2,3-dihydro-1H-imidazo[4,5-b]pyridine-7-yl)oxy)phenyl)-2-(trifluoromethyl)benzamide |
| Example 26 | 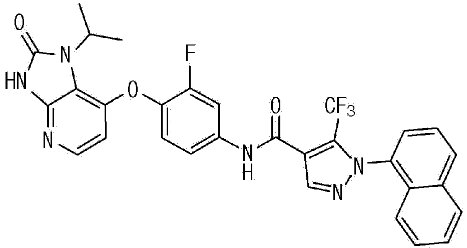 | N-(3-fluoro-4-((1-isopropyl-2-oxo-2,3-dihydro-1H-imidazo[4,5-b]pyridine-7-yl)oxy)phenyl)-1-(naphthalene-1-yl)-5-(trifluoromethyl)-1H-pyrazole-4-carboxamide |
| Example 27 | 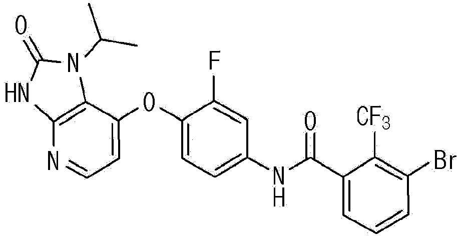 | 3-bromo-N-(3-fluoro-4-((1-isopropyl-2-oxo-2,3-dihydro-1H-imidazo[4,5-b]pyridine-7-yl)oxy)phenyl)-2-(trifluoromethyl)benzamide |
| Example 28 | 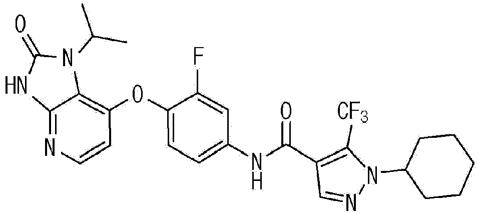 | 1-cyclohexyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide |
| Example 29 | 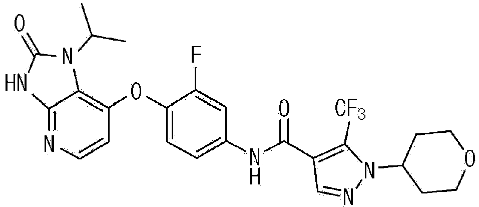 | N-(3-fluoro-4-((1-isopropyl-2-oxo-2,3-dihydro-1H-imidazo[4,5-b]pyridine-7-yl)oxy)phenyl)-1-(tetrahydro-2H-pyran-4-yl)-5-(trifluoromethyl)-1H-pyrazole-4-carboxamide |
| Example 30 | 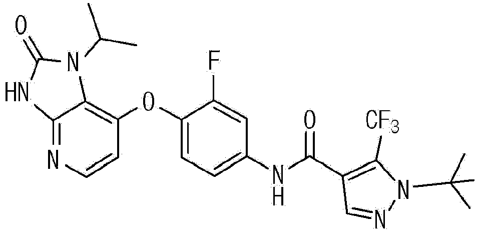 | 1-(tert-butyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide |
| Example 31 | 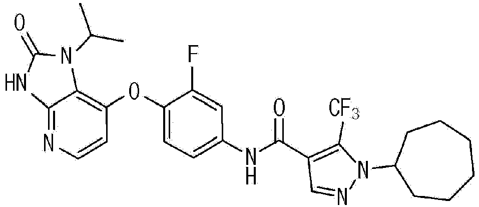 | 1-cycloheptyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide |
| Example 32 | 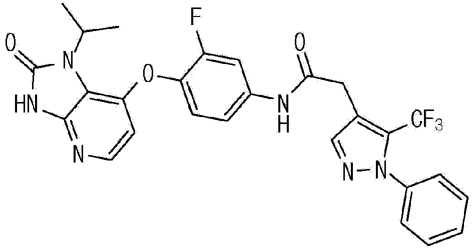 | N-(3-fluoro-4-((1-isopropyl-2-oxo-2,3-dihydro-1H-imidazo[4,5-b]pyridine-7-yl)oxy)phenyl)-2-(1-phenyl-5-(trifluoromethyl)-1H-pyrazol-4-yl)acetamide |
| Example 33 | 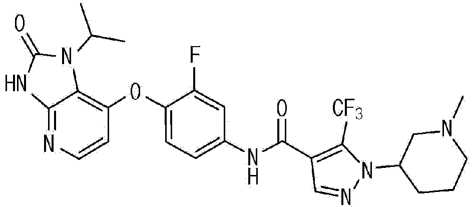 | N-(3-fluoro-4-((1-isopropyl-2-oxo-2,3-dihydro-1H-imidazo[4,5-b]pyridine-7-yl)oxy)phenyl)-1-(1-methylpiperidine-3-yl)-5-(trifluoromethyl)-1H-pyrazole-4-carboxamide |
| Example 34 | 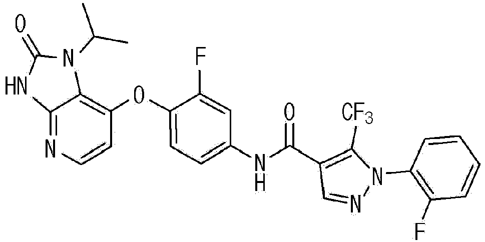 | N-(3-fluoro-4-((1-isopropyl-2-oxo-2,3-dihydro-1H-imidazo[4,5-b]pyridine-7-yl)oxy)phenyl)-1-(2fluorophenyl)-5-(trifluoromethyl)-1H-pyrazole-4-carboxamide |
| Example 35 | 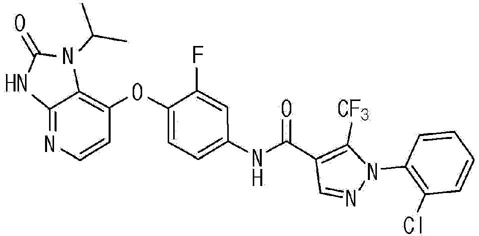 | 1-(2chlorophenyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide, |
| Example 36 | 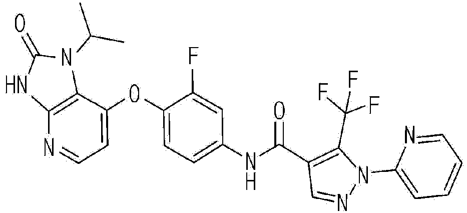 | 1-(2,6-difluorophenyl)-N-(3-fluoro-4-((1-isopropyl-2-oxo-2,3-dihydro-1H-imidazo[4,5- b]pyridine-7-yl)oxy)phenyl)-5-(trifluoromethyl)-1H-pyrazole-4-carboxamide |
| Example 37 | 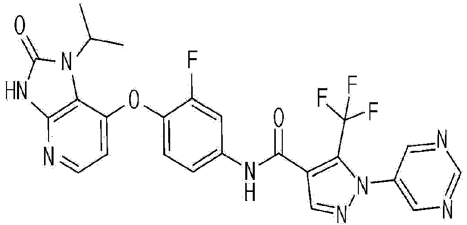 | N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-pyrazole-4-carboxamide |
| Example 38 | 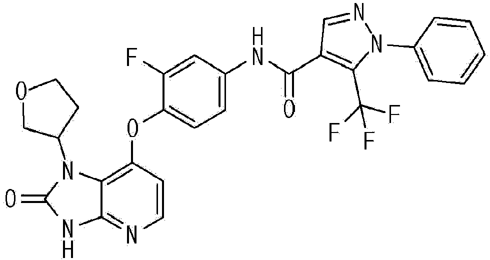 | N-(3-fluoro-4-((2-oxo-1-(tetrahydrofuran-3 -yl)-2,3 - dihydro-1H-imidazo[4,5-b]pyridine-7-yl)oxy)phenyl)-1-phenyl-5-(trifluoromethyl)-1H-pyrazole-4-carboxamide |
| Example 39 | 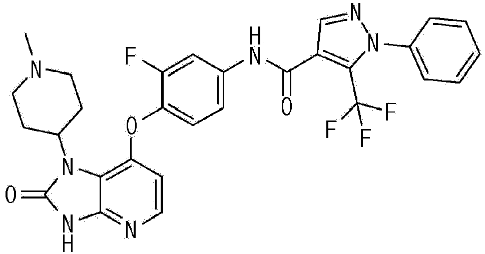 | N-(3-fluoro-4-((1-(1-methylpiperidine-4-yl)-2-oxo-2,3-dihydro-1H-imidazo[4,5-b]pyridine-7-yl)oxy)phenyl)-1-phenyl-5-(trifluoromethyl)-1H-pyrazole-4-carboxamide |
| Example 40 | 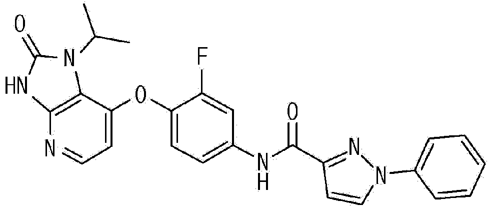 | N-(3-fluoro-4-((1-isopropyl-2-oxo-2,3-dihydro-1H-imidazo[4,5-b]pyridine-7-yl)oxy)phenyl)-1-phenyl-1H-pyrazole-3-carboxamide |
| Example 41 | 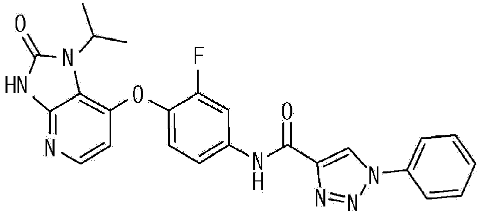 | N-(3-fluoro-4-((1-isopropyl-2-oxo-2,3-dihydro-1H-imidazo[4,5-b]pyridine-7-yl)oxy)phenyl)-1-phenyl-1H-1,2,3-triazole-4-carboxamide |
| Example 42 | 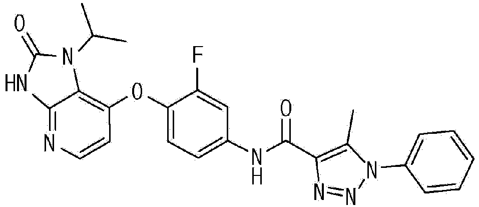 | N-(3-fluoro-4-((1-isopropyl-2-oxo-2,3-dihydro-1H-imidazo[4,5-b]pyridine-7-yl)oxy)phenyl)-5-methyl-1-phenyl-1H-1,2,3-triazole-4-carboxamide |
| Example 43 | 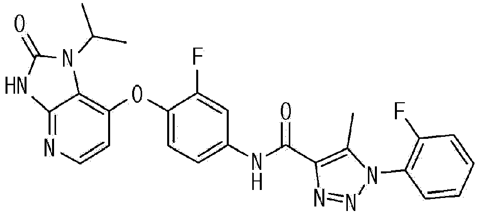 | N-(3-fluoro-4-((1-isopropyl-2-oxo-2,3-dihydro-1H-imidazo[4,5-b]pyridine-7-yl)oxy)phenyl)-1-(2-fluorophenyl)-1H-1,2,3-triazole-4-carboxamide |

In the present invention, the "pharmaceutically acceptable salt" includes a salt commonly used to form an alkali metal salt and to form an additional salt of a free acid or a free base. The properties of such salts are not important, but the salts should be pharmaceutically acceptable. A pharmaceutically acceptable acid addition salt suitable for a compound of Formula 1 may be prepared from an inorganic acid or an organic acid. Examples of the inorganic acid may include a hydrochloric acid, a hydrobromic acid, a hydroiodic acid, a nitric acid, a carbonic acid, a sulfuric acid, and a phosphoric acid. A suitable organic acid may be selected from an organic acid of aliphatic, cycloaliphatic, aromatic, arylaliphatic, heterocyclic, carboxylic, and sulfonic classes, and examples thereof may include a formic acid, an acetic acid, an adipic acid, a butyric acid, a propionic acid, a succinic acid, a glycolic acid, a gluconic acid, a lactic acid, a malic acid, a tartaric acid, a citric acid, an ascorbic acid, a glucuronic acid, a maleic acid, a fumaric acid, a pyruvic acid, an aspartic acid, a glutamic acid, a benzoic acid, an anthranilic acid, a mesylic acid, a 4-hydroxybenzoic acid, a phenylacetic acid, a mandelic acid, an embonic acid (pamoic acid), a methanesulfonic acid, an ethanesulfonic acid, an ethanedisulfonic acid, a benzenesulfonic acid, a pantothenic acid, a 2-hydroxyethanesulfonic acid, a toluenesulfonic acid, a sulfanilic acid, a cyclohexylaminosulfonic acid, a camphoric acid, a camphorsulfonic acid, a digluconic acid, a cyclopentanepropionic acid, a dodecylsulfonic acid, a glucoheptanoic acid, a glycerophosphonic acid, a heptanoic acid, a hexanoic acid, a 2-hydroxy-ethanesulfonic acid, a nicotinic acid, a 2-naphthalenesulfonic acid, an oxalic acid, a palmoic acid, a pectinic acid, a persulfuric acid, a 2-phenylpropionic acid, a picric acid, a pivalic acid, a propionic acid, a succinic acid, a tartaric acid, a thiocyanic acid, a mesylic acid, an undecanoic acid, a stearic acid, an algenic acid, a β-hydroxybutyric acid, a salicylic acid, a galactaric acid, and a galacturonic acid. A pharmaceutically acceptable base addition salt suitable for a compound of Formula 1 may be a metal salt, for example, a salt prepared from aluminum, calcium, lithium, magnesium, potassium, sodium, or zinc, or a salt prepared from an organic base including substituted amine, including primary, secondary, or tertiary amine and cyclic amine, for example, caffeine, arginine, diethylamine, N-ethyl piperidine, istidine, glucamine, isopropylamine, lysine, morpholine, N-ethyl morpholine, piperazine, piperidine, triethylamine, or trimethylamine. These salts may be prepared, from a corresponding compound of the present invention, by a typical method such as reacting a suitable acid or base with a compound of Formula 1. When a basic group and an acidic group are present in the same molecule, the compound of Formula 1 may also form an internal salt. In the present invention, the "solvate" may include a hydrate, a solvate with an organic solvent such as methanol, ethanol, 2-propanol, 1,2-propanediol, 1,3-propanediol, n-butanol, 1,4-butanediol, tert-butanol, an acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and a mixture thereof.

According to another aspect, the present invention provides a pharmaceutical composition including the novel compound of Formula 1 defined above or the pharmaceutically acceptable salt thereof, and a pharmaceutically acceptable carrier.

The pharmaceutical composition may be useful in the prevention or treatment of a protein kinase-mediated disease, but the use of the present invention is not limited to these diseases.

In one embodiment, the pharmaceutical composition may be useful in the prevention or treatment of a RON-mediated disease.

The pharmaceutical composition according to the present invention includes a therapeutically effective amount of the compound of Formula 1 above or the pharmaceutically acceptable salt thereof.

In one embodiment, the disease may be a cancer selected from the group consisting of lung cancer, breast cancer, colorectal cancer, kidney cancer, pancreatic cancer, head cancer, cervical cancer, hereditary papillary renal cell carcinoma, pediatric hepatocellular carcinoma, and gastric cancer, but is not limited thereto.

In another embodiment, the disease may be an immune disease selected from the group consisting of an inflammatory disorder, a cardiovascular disease, a virus-induced disease, a circulatory disease, a fibro-proliferative disease, and pain sensation, but is not limited thereto.

For the treatment of the above diseases, the pharmaceutical composition may be administered to a subject in need of prevention or treatment of the above diseases.

In the present specification, the term "prevention" means reducing the risk of contracting a disease or disorder, and it means any action that suppresses or delays the onset of a disease by preventing the progression of more than 1 type of clinical symptoms of the disease in a subject who is exposed to or susceptible to the disease but does not yet have the disease or exhibit symptoms of the disease.

In the present specification, the term "treatment" means alleviating a disease or disorder, and it means any action that reduces or beneficially alters symptoms of the disease by stopping or reducing the progression of the disease or one or more clinical symptoms thereof.

In the present specification, the term "carrier" means a compound that facilitates the introduction of a compound into cells or tissues. The pharmaceutical composition may be prepared in a unit dose form by formulation using a pharmaceutically acceptable carrier, or may be prepared by being introduced into a multi-dose container. At this time, the formulation may be in the form of a solution, suspension, or emulsion in an oil or aqueous medium, or may be in the form of an extract, powder, granule, tablet, capsule, or gel (e.g., hydrogel), and may additionally contain a dispersant or a stabilizer.

In addition, the compound represented by Formula 1 or the pharmaceutically acceptable salt thereof included in the pharmaceutical composition may be carried in a pharmaceutically acceptable carrier such as colloidal suspension, powder, saline solution, lipids, liposomes, microspheres, or nanospherical particles. The above may form or be associated with a complex with transport means, and may be carried in vivo using a transport system known in the art such as lipids, liposomes, microparticles, gold, nanoparticles, polymers, condensation reactors, polysaccharides, polyamino acids, dendrimers, saponins, adsorption enhancing substances, or fatty acids.

In addition, the pharmaceutically acceptable carrier may include lactose, dextrose, sucrose, sorbitol, mannitol, starch, acacia, rubber, calcium phosphate, alginate, gelatin, calcium silicate, microcrystalline cellulose, polyvinyl pyrrolidone, cellulose, water, syrup, methyl cellulose, methylhydroxybenzoate, propyl hydroxybenzoate, talc, magnesium stearate, mineral oil, and the like, which are typically used in formulation, but is not limited thereto. In addition, a lubricant, a wetting agent, a sweetening agent, a flavoring agent, an emulsifying agent, a suspending agent, a preservative, and the like may be additionally included in addition to the above components.

The pharmaceutical composition according to the present invention may be administered orally or parenterally at the time of clinical administration and may be used in the form of a general pharmaceutical formulation. That is, the pharmaceutical composition of the present invention may be administered in various formulations of oral and parenteral at the time of actual clinical administration, and when formulated, is prepared using a diluent or an excipient such as a filler, an extender, a binder, a wetting agent, a disintegrant, a surfactant, and the like, which are commonly used. Solid formulations for oral administration include tablets, pills, powders, granules, capsules, and the like, and such solid formulations are prepared by mixing at least one excipient, for example, starch, calcium carbonate, sucrose or lactose, gelatin, and the like in a herbal medicine extract or a herbal medicine fermented product. In addition, lubricants such as magnesium stearate talc are also used in addition to simple excipients. Liquid formulations for oral administration may include suspensions, internal liquids, emulsions, syrups, and the like, and in addition to water and liquid paraffin, which are commonly used simple diluents, various excipients such as a wetting agent, a sweetening agent, a flavoring agent, a fragrance, a preservative, and the like may be included. Formulations for parenteral administration include sterile aqueous solutions, non-aqueous solvents, suspending agents, emulsions, freeze-dried formulations, and suppositories. Examples of non-aqueous solvents and suspensions may include propylene glycol, polyethylene glycol, vegetable oils such as olive oil, and injectable esters such as ethyl oleate. As base materials of suppositories, witepsol, macrogol, tween 61, cacao butter, laurin fat, glycerol, gelatin, and the like may be used.

When the pharmaceutical composition is administered for clinical purposes, the effective dosage of the compound of Formula 1 or the pharmaceutically acceptable salt thereof may vary depending on factors such as a formulation method, an administration mode, a patient's age, weight, sex, pathological conditions, and diet, administration time, administration route, excretion rate, drug mixing, and response sensitivity, but in general, the pharmaceutical composition may be administered 0.01 to 20 mg/day, preferably 1 to 10 mg/day, per 1 kg of body weight of an adult patient, and may be administered in installments several times per day, preferably 2 to 3 times per day, at regular time intervals at the discretion of a doctor or pharmacist.

In another aspect, the present invention provides a method for treating protein kinase-mediated diseases, RON-mediated diseases in particular, the method including administering to a subject a therapeutically effective amount of the compound of Formula 1 above or the pharmaceutically acceptable salt thereof.

In another aspect, the present invention provides a method for inhibiting the activity of a RON receptor, the method including administering to a subject a therapeutically effective amount of the compound of Formula 1 above or the pharmaceutically acceptable salt thereof.

In the present specification, the term "therapeutically effective amount" means the amount of each formulation that achieves the purpose of reducing the severity of a disease and frequency of onset thereof during the treatment by each formulation itself, but avoids harmful side effects typically associated with other therapies. For example, an effective agent for tumor treatment is effective in prolonging the survival period of a patient, inhibiting the growth of rapidly proliferating cells associated with a tumor, or in regressing the tumor.

Hereinafter, example compounds of the present invention may be prepared according to the following method, but compounds of the present invention are not limited to the preparation method.

### Preparation Example 1. 7-(4-amino-2-fluorophenoxy)-1-isopropyl-1,3-dihydro-2H-imidazo [4,5-b] pyridine-2-one

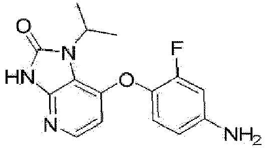

Hereinafter, a compound of Preparation Example 1 was prepared according to Reaction Formula 1 below.

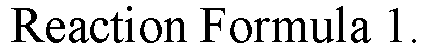

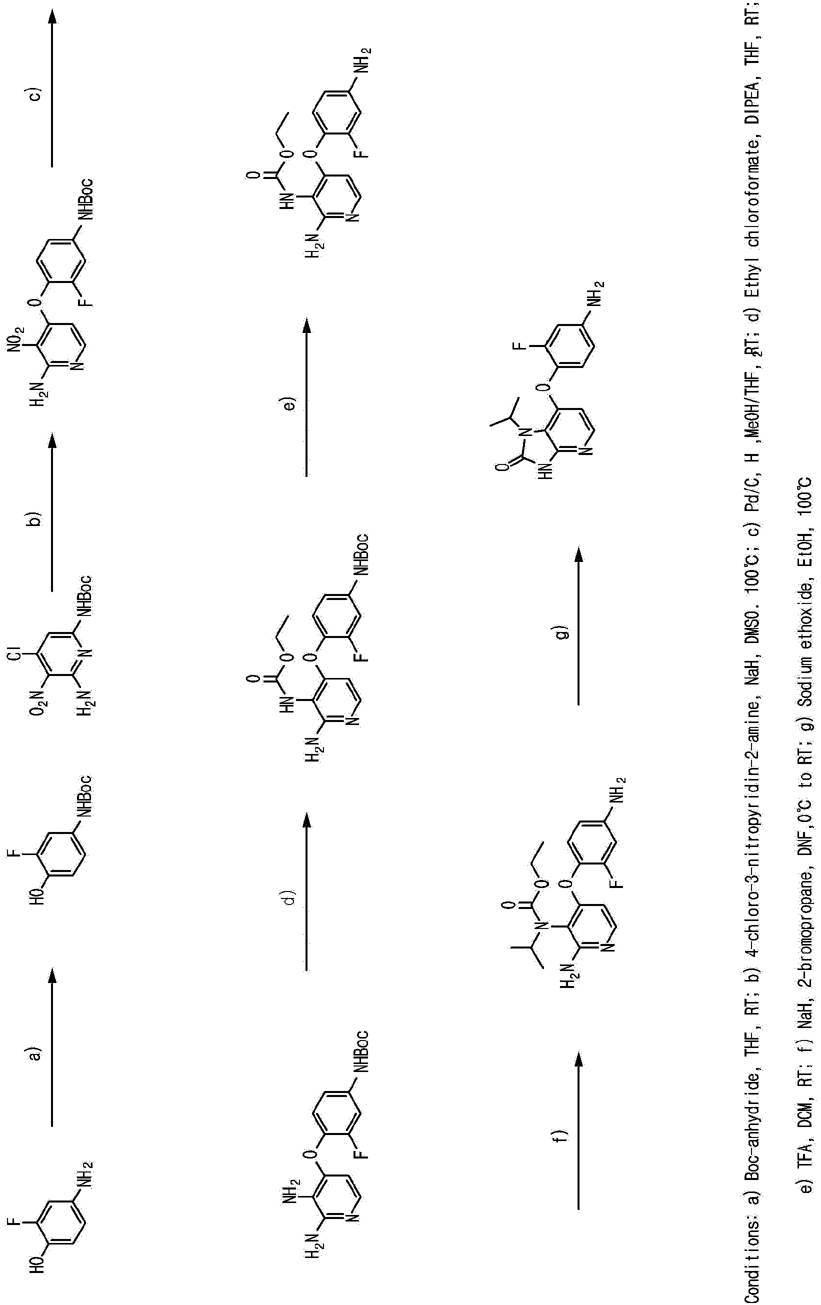

### Step 1) Tert-butyl (3-fluoro-4-hydroxyphenyl)carbamate

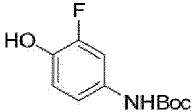

4-amino-2-fluorophenol (10 g, 79 mmol) was dissolved in THF (100 mmol) and then Boc-anhydride (34.3 g, 158 mmol) was added thereto, and the mixture was stirred at room temperature for 12 hours. The reaction mixture was concentrated, and the residue was purified by column chromatography to obtain a title compound (13 g, yield: 72%).

MS m/z : 228[M+H].

### Step 2) Tert-butyl (4-((2-amino-3-nitropyridine-4-yl)oxy)-3-fluorophenyl)carbamate

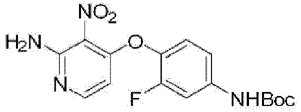

The compound tert-butyl (3-fluoro-4-hydroxyphenyl)carbamate (12.5 g, 55 mmol) obtained in Step 1 above and 4-chloro-3-nitropyridine-2-amine (9.55 g, 55 mmol) were dissolved in 60 mL of DMSO, and then NaH (2.64 g, 66 mmol) was added thereto at 0°C, and the mixture was stirred at 100°C for 12 hours. The reaction mixture was diluted with ethyl acetate, washed with water and brine, and then concentrated. The residue was purified by column chromatography to obtain a title compound (15 g, yield: 75%).

MS *m*/*z* : 365[M+H].

### Step 3) Tert-butyl (4-((2,3-diaminopyridine-4-yl)oxy)-3-fluorophenyl)carbamate

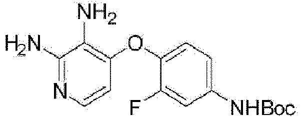

The compound tert-butyl (4-((2-amino-3-nitropyridine-4-yl)oxy)-3-fluorophenyl)carbamate obtained in Step 2 above was dissolved in a methanol/THF mixed solvent at room temperature, and then Pd/C was added thereto, and the mixture was stirred under hydrogen conditions for 12 hours. The reaction mixture was filtered through a celite filter and then concentrated. The residue was purified by column chromatography to obtain a title compound (11.5 g, yield: 84%).

MS *m*/*z* : 335[M+H].

### Step 4) Tert-butyl (4-((2-amino-3-((ethoxycarbonyl)amino)pyridine-4-yl)oxy)-3-fluorophenyl)carbamate

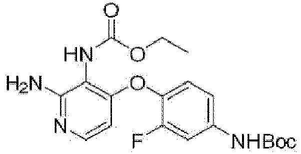

The compound tert-butyl (4-((2,3-diaminopyridine-4-yl)oxy)-3-fluorophenyl)carbamate (10 g, 29.9 mmol) obtained in Step 3 above and pyridine (4.73 g, 59.8 mmol) were dissolved in THF, and then ethyl chloroformate (3.57 g, 32.9 mmol) was slowly added thereto at 0°C. The mixture was stirred for 12 hours, and then the reaction mixture was diluted with ethyl acetate, washed with water and brine, and then concentrated. The residue was purified by column chromatography to obtain a title compound (4.5 g, yield: 37%).

MS *m*/*z* : 407[M+H].

### Step 5) Ethyl (2-amino-4-(4-amino-2-fluorophenoxy)pyridine-3-yl)carbamate

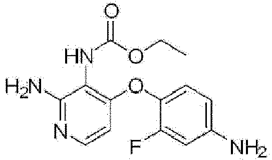

The compound tert-butyl (4-((2-amino-3-((ethoxycarbonyl)amino)pyridine-4-yl)oxy)-3-fluorophenyl)carbamate (3.5 g, 8.61 mmol) obtained in Step 4 above was dissolved in a TFA/DCM 1:5 mixed solvent and stirred at room temperature for 5 hours. The reaction mixture was diluted with ethyl acetate, washed with water and brine, and purified by column chromatography to obtain a title compound (1.9 g, yield: 72%).

MS *m*/*z:* 307[M+H].

### Step 6) Ethyl (2-amino-4-(4-amino-2-fluorophenoxy)pyridine-3-yl)(isopropyl)carbamate

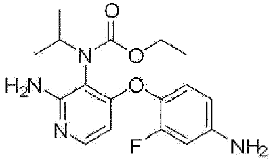

The compound ethyl (2-amino-4-(4-amino-2-fluorophenoxy)pyridine-3-yl)carbamate (1.9 g, 6.2 mmol) obtained in Step 5 above was dissolved in DMF, and then NaH (0.273 g, 6.82 mmol) was added thereto at 0°C, followed slowly adding 2-bromopropane (0.84 g, 6.82 mmol) thereto. The mixture was stirred for 12 hours, and then the reaction mixture was diluted with ethyl acetate, washed with water and brine, and then concentrated. The residue was purified by column chromatography to obtain a title compound (1.25 g, yield: 58%).

MS *m*/*z* : 349[M+H].

### Step 7) 7-(4-amino-2-fluorophenoxy)-1-isopropyl-1,3-dihydro-2H-imidazo[4,5-b]pyridine-2-one

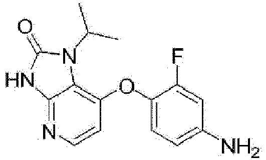

The compound ethyl (2-amino-4-(4-amino-2-fluorophenoxy)pyridine-3-yl)(isopropyl)carbamate (1.25 g, 3.59 mmol) obtained in Step 6 above and sodium ethoxide (0.488 g, 7.18 mmol) were dissolved in ethanol and the mixture was stirred under a microwave condition and 120°C for 4 hours. Water was added to the reaction mixture crystallize the mixture, thereby obtaining a title compound (1.0 g, yield: 92%).

¹H NMR (500 MHz, MeOH-d₄) δ 7.76 (d, 1H), 7.03 (t, 1H), 6.62 (dd, 1H), 6.57(dd, 1H), 6.33 (d, 1H), 5.02 (m, 1H), 1.58 (s, 3H), 1.07 (s, 3H);

MS *m*/*z* : 303[M+H]

### Preparation Example 2. 7-(4-amino-2-fluorophenoxy)-1-ethyl -1,3-dihydro-2H-imidazo[4,5-b]pyridine-2-one

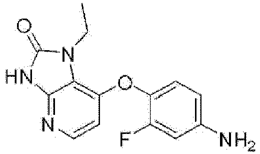

A title compound (63 mg, yield: 56%) was obtained in a manner similar to that of Preparative Example 1 by using ethyl iodide instead of 2-bromopropane in Step 6 of Preparation Example 1.

MS *m*/*z :* 289[M+H].

### Preparation Example 3. 7-(4-amino-2-fluorophenoxy)-1-methyl-1,3-dihydro-2H-imidazo[4,5-b]pyridine-2-one

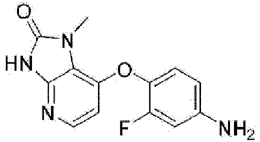

A title compound (90 mg, yield: 74%) was obtained in a manner similar to that of Preparative Example 1 by using methyl iodide instead of 2-bromopropane in Step 6 of Preparation Example 1.

MS *m*/*z* : 275[M+H].

### Preparation Example 4. 7-(4-amino-2-fluorophenoxy)-1-cyclopentyl-1,3-dihydro-2H-imidazo[4,5-b]pyridine-2-one

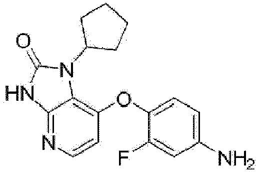

A title compound (150 mg, yield: 86%) was obtained in a manner similar to that of Preparative Example 1 by using cyclopentyl bromide instead of 2-bromopropane in Step 6 of Preparation Example 1.

MS *m*/*z* : 329[M+H].

### Preparation Example 5. 7-(4-amino-2-fluorophenoxy)-1,3-dihydro-2H-imidazo[4,5-b]pyridine-2-one

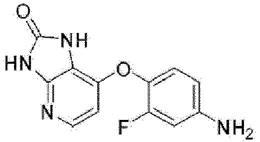

Hereinafter, a compound of Preparation Example 5 was prepared according to Reaction Formula 2 below.

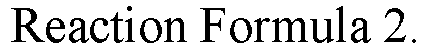

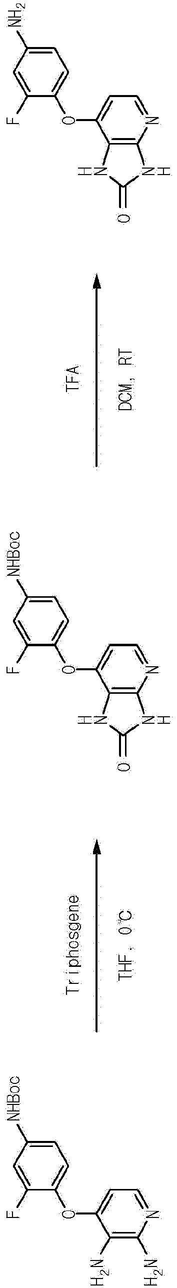

### Step 1) Tert-butyl(3-fluoro-4-((2-oxo-2,3-dihydro-1H-imidazo[4,5-b]pyridine-7-yl)oxy)phenyl)carbamate

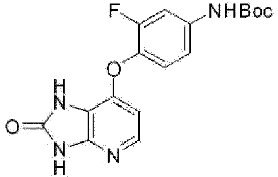

Tert-butyl (4-((2,3-diaminopyridine-4-yl)oxy)-3-fluorophenyl)carbamate (250 mg, 0.75 mmol) was dissolved in THF, and the mixture was cooled to 0°C. Triphosgene (225 mg, 0.75 mmol) was added thereto, and the mixture was stirred at 0°C for 30 minutes. Saturated NaHCO₃ was added thereto, and the mixture was extracted 3 times with ethyl acetate. The organic layer was washed with brine and concentrated, and the residue was purified by column chromatography to obtain a title compound (85 mg, yield: 32%).

MS *m*/*z :* 361[M+H].

### Step 2) 7-(4-amino-2-fluorophenoxy)-1,3-dihydro-2H-imidazo[4,5-b]pyridine-2-one

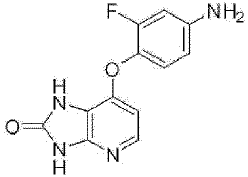

The compound tert-butyl(3-fluoro-4-((2-oxo-2,3-dihydro-1H-imidazo[4,5-b]pyridine-7-yl)oxy)phenyl)carbamate (80 mg, 0.22 mmol) obtained in Step 1 above was dissolved in 3 ml of DCM, and 1.5 ml of TFA was added thereto. The reaction mixture was stirred at room temperature for 2 hours. The reaction mixture was concentrated, diluted with ethyl acetate, and washed with saturated NaHCO₃. The organic layer was washed with brine and concentrated to obtain a title compound (40 mg, yield: 69%).

MS *m*/*z :* 261[M+H].

### Preparation Example 6. 1-(2-fluorophenyl)-5-(trifluoromethyl)-1H-pyrazole-4-carboxylic acid

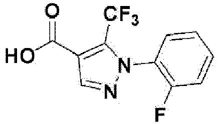

Hereinafter, a compound of Preparation Example 6 was prepared according to Reaction Formula 3 below.

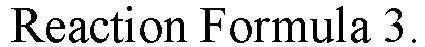

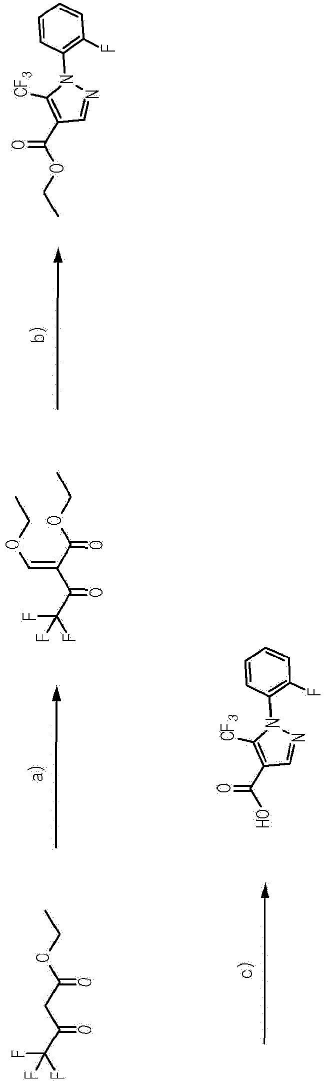

### Step 1) Ethyl 2-(ethoxymethylene)-4,4,4-trifluoro-3-oxobutanoate

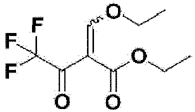

Ethyl 4,4,4-trifluoro-3-oxobutinoate (2.0 g, 10.9 mmol) and triethoxymethane (2.0 g, 14.1 mmol) were dissolved in acetic anhydride (3.3 g, 32.6 mmol), and the mixture was stirred at 130°C for 4 hours. The reaction mixture was concentrated to obtain a title compound (2.0 g, yield: 77%).

MS *m*/*z :* 241[M+H].

### Step 2) Ethyl 1-(2-fluorophenyl)-5-(trifluoromethyl)-1H-pyrazole-4-carboxylate

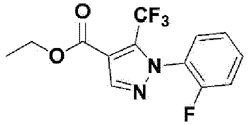

The compound ethyl 2-(ethoxymethylene)-4,4,4-trifluoro-3-oxobutanoate (0.5 g, 2.1 mmol) obtained in Step 1 above and (2-fluorophenyl)hydrazine (0.263 g, 2.1 mmol) were dissolved in 7 ml of ethanol and the mixture was stirred at 70°C for 4 hours. The reaction mixture was extracted with ethyl acetate and water and concentrated. The residue was purified by column chromatography to obtain a title compound (0.38 g, yield: 60%).

MS *m*/*z* : 303[M+H].

### Step 3) 1-(2-fluorophenyl)-5-(trifluoromethyl)-1H-pyrazole-4-carboxylic acid

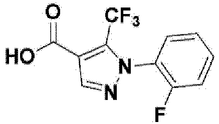

The compound ethyl 1-(2-fluorophenyl)-5-(trifluoromethyl)-1H-pyrazole-4-carboxylate (0.38 g, 1.3 mmol) obtained in Step 2 above was dissolved in 4 ml ethanol, and then 2 ml of a 6N sodium hydroxide solution was added thereto at room temperature, and the mixture was stirred for 1 hour. The solid formed by adding ice water to the reaction product was filtered to obtain a title compound (0.18 g, yield: 52%).

MS *m*/*z* : 275[M+H].

### Example 1. N-(3-fluoro-4-((2-oxo-2,3-dihydro-1H-imidazo[4,5-b]pyridine-7-yl)oxy)phenyl)-1-phenyl-5-(trifluoromethyl)-1H-imidazole-4-carboxamide

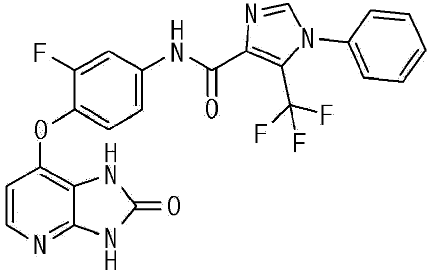

Hereinafter, a compound of Examples 1 was prepared according to Reaction Formula 4 below.

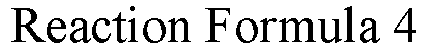

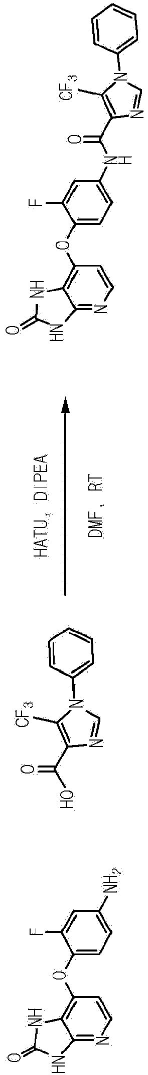

The compound 7-(4-amino-2-fluorophenoxy)-1H-1,3-dihydro-2H-imidazo[4,5-b]pyridine-2-one (20 mg, 0.07 mmol) obtained in Preparation Example 5 above, 1-phenyl-5-(trifluoromethyl)-1H-imidazole-4-carboxylic acid (20 mg, 0.08 mmol), and HATU (40 mg, 0.10 mmol) were dissolved in 2 mL of DMF, and then DIPEA (17 µl, 0.10 mmol) was added thereto, and the mixture was stirred at room temperature for 12 hours. The reaction mixture was diluted with ethyl acetate, washed with water and brine, and then concentrated. The residue was purified by prep-HPLC (0.1% formic acid in water/acetonitrile) to obtain a title compound (11 mg, yield: 30%).

¹H NMR (500 MHz, DMSO-d₆) δ 11.30 (brs, 1H), 10.77 (s, 1H), 8.35 (s, 1H), 8.04 (m, 1H), 7.78 (d, 1H), 7.72 (m, 1H), 7.60 (m, 3H), 7.39 (t, 1H), 6.37 (d, 1H); MS *m*/*z :* 499[M+H]

### Example 2. N-(3-fluoro-4-((2-oxo-2,3-dihydro-1H-imidazo[4,5-b]pyridine-7-yl)oxy)phenyl)-1-phenyl-5-(trifluoromethyl)-1H-pyrazole-4-carboxamide

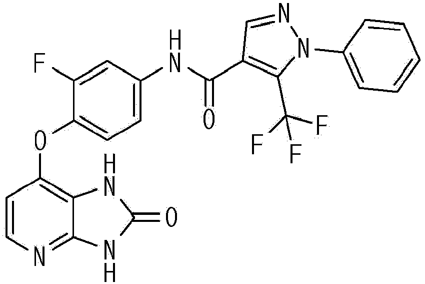

A title compound (9 mg, yield: 28%) was obtained in a manner similar to that of Example 1 by using 1-phenyl-5-(trifluoromethyl)-1H-pyrazole-4-carboxylic acid instead of 1-phenyl-5-(trifluoromethyl)-1H-imidazole-4-carboxylic acid according to Example 1.

¹H NMR (500 MHz, DMSO-d₆) δ 10.84 (s, 1H), 8.34 (s, 1H), 7.93 (dd, 1H), 7.78 (d, 1H), 7.64 (m, 3H), 7.56 (m, 3H), 7.40 (t, 1H), 6.38 (d, 1H); MS *m*/*z* : 499[M+H]

### Example 3. N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide

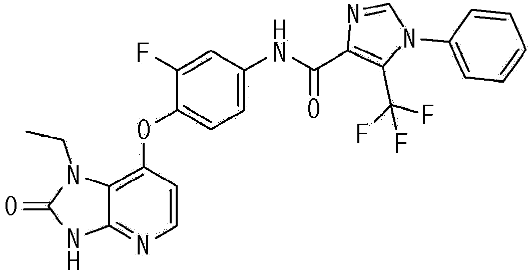

A title compound (35 mg, yield: 64%) was obtained in a manner similar to that of Example 1 by using 7-(4-amino-2-fluorophenoxy)-1-ethyl-1,3-dihydro-2H-imidazo[4,5-b]pyridine-2-one obtained in Preparation Example 2 instead of 7-(4-amino-2-fluorophenoxy)-1H-1,3-dihydro-2H-imidazo[4,5-b]pyridine-2-one according to Example 1.

¹H NMR (500 MHz, DMSO-d₆) δ 11.64 (brs, 1H), 10.74 (s, 1H), 8.31 (s, 1H), 8.01 (d, 1H), 7.98 (d, 1H), 7.75 (m, 1H), 7.59 (m, 5H), 7.41 (t, 1H), 6.36 (d, 1H), 3.97 (q, 2H), 1.26 (t, 3H); MS *m*/*z :* 527[M+H].

### Example 4. N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide

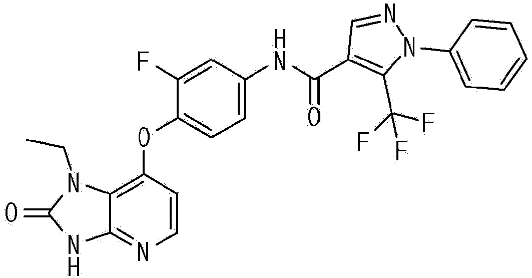

A title compound (33 mg, yield: 60%) was obtained in a manner similar to that of Example 3 by using 1-phenyl-5-(trifluoromethyl)-1H-pyrazole-4-carboxylic acid instead of 1-phenyl-5-(trifluoromethyl)-1H-imidazole-4-carboxylic acid according to Example 3.

¹H NMR (500 MHz, DMSO-d₆) δ 11.62 (brs, 1H), 10.81 (s, 1H), 8.30 (s, 1H), 7.90 (d, 1H), 7.77 (d, 1H), 7.59 (m, 3H), 7.52 (m, 3H), 7.43 (t, 1H), 6.37 (d, 1H), 3.97 (q, 2H), 1.26 (t, 3H); MS *m*/*z* : 527[M+H].

### Example 5. N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-imidazole-4-carboxamide

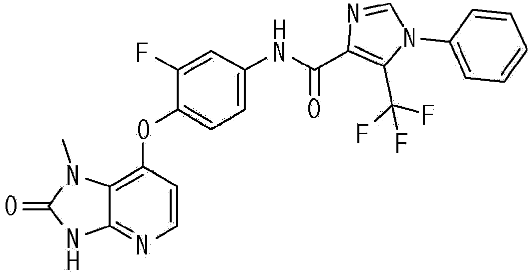

A title compound (15 mg, yield: 26%) was obtained in a manner similar to that of Example 1 by using 7-(4-amino-2-fluorophenoxy)-1-methyl-1,3-dihydro-2H-imidazo[4,5-b]pyridine-2-one obtained in Preparation Example 3 instead of 7-(4-amino-2-fluorophenoxy)-1-H-1,3-dihydro-2H-imidazo[4,5-b]pyridine-2-one according to Example 1.

¹H NMR (500 MHz, DMSO-d₆) δ 11.63 (brs, 1H), 10.73 (s, 1H), 8.31 (s, 1H), 7.97 (d, 1H), 7.76 (d, 1H), 7.63 (m, 1H), 7.48 (m, SH), 7.38 (t, 1H), 6.38 (d, 1H), 3.47 (s, 3H); MS *m*/*z* : 513[M+H].

### Example 6. N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-pyrazole-4-carboxamide

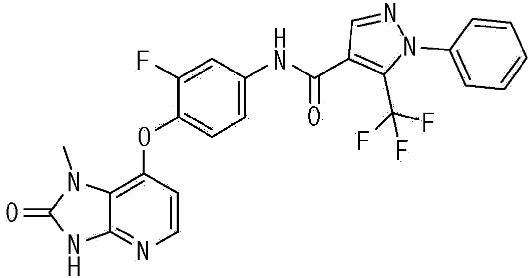

A title compound (15 mg, yield: 21%) was obtained in a manner similar to that of Example 5 by using 1-phenyl-5-(trifluoromethyl)-1H-pyrazole-4-carboxylic acid instead of 1-phenyl-5-(trifluoromethyl)-1H-imidazole-4-carboxylic acid according to Example 5.

¹H NMR (500 MHz, DMSO-d₆) δ 11.64 (brs, 1H), 10.80 (s, 1H), 8.30 (s, 1H), 7.88 (d, 1H), 7.76 (d, 1H), 7.59 (m, 3H), 7.50 (m, 3H), 7.40 (t, 1H), 6.38 (d, 1H), 3.46 (s, 3H); MS *m*/*z* : 513[M+H].

### Example 7. N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-pyrazole-4-carboxamide

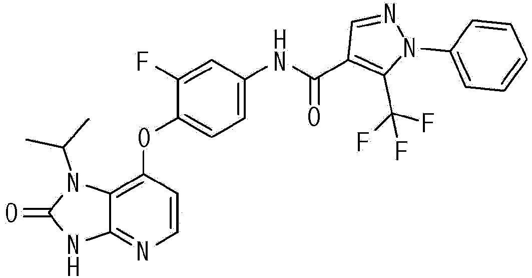

A title compound (10 mg, yield: 11%) was obtained in a manner similar to that of Example 1 by using 7-(4-amino-2-fluorophenoxy)-1-isopropyl-1,3-dihydro-2H-imidazo[4,5-b]pyridine-2-one obtained in Preparation Example 1 instead of 7-(4-amino-2-fluorophenoxy)-1-H-1,3-dihydro-2H-imidazo[4,5-b]pyridine-2-one according to Example 1.

¹H NMR (500 MHz, MeOH-d₄) δ 8.13 (s, 1H), 7.89 (m, 2H), 7.58 (m, 6H), 7.36 (t, 1H), 6.45 (d, 1H), 5.01 (m, 1H), 1.57 (d, 6H); MS *m*/*z* : 541[M+H].

### Example 8. N-(3-fluoro-4-((1-isopropyl-2-oxo-2,3-dihydro-1H-imidazo[4,5-b]pyridine-7-yl)oxy)phenyl)-1-phenyl-5-(trifluoromethyl)-1H-imidazole-4-carboxamide

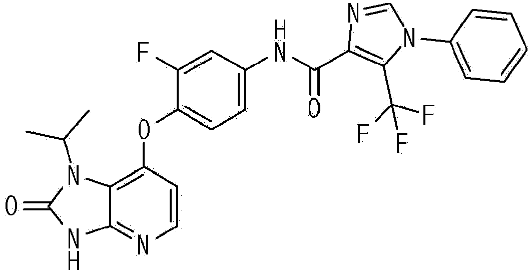

A title compound (36 mg, yield: 50%) was obtained in a manner similar to that of Example 7 by using 1-phenyl-5-(trifluoromethyl)-1H-imidazole-4-carboxylic acid instead of 1-phenyl-5-(trifluoromethyl)-1H-pyrazole-4-carboxylic acid according to Example 7.

¹H NMR (500 MHz, DMSO-d₆) δ 11.68 (brs, 1H), 10.78 (s, 1H), 8.35 (s, 1H), 8.06 (m, 1H), 7.81 (d, 1H), 7.76 (m, 1H), 7.62 (m, 5H), 7.42 (t, 1H), 6.42 (d, 1H), 4.90 (m, 1H), 1.49 (d, 6H), 1.47 (s, 3H); MS *m*/*z* : 541[M+H].

### Example 9. 5-ethyl-N-(3-fluoro-4-((2-oxo-2,3-dihydro-1H-imidazo[4,5-b]pyridine-7-yl)oxy)phenyl)-1-phenyl-1H-pyrazole-4-carboxamide

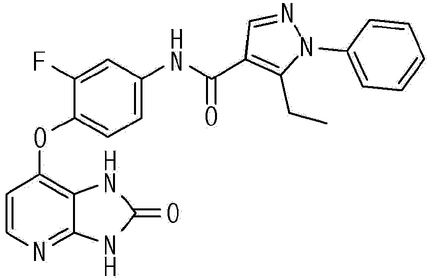

A title compound (26 mg, yield: 53%) was obtained in a manner similar to that of Example 1 by using 5-ethyl-1-phenyl-1H-pyrazole-4-carboxylic acid instead of 1-phenyl-5-(trifluoromethyl)-1H-imidazole-4-carboxylic acid according to Example 1.
¹H NMR (500 MHz, DMSO-d₆) δ 11.41 (brs, 1H), 11.23 (brs, 1H), 10.14(s, 1H), 8.32 (s, 1H), 7.98 (d, 1H), 7.78 (d, 1H), 7.62 (m, 4H), 7.51 (m, 2H), 7.38 (t, 1H), 6.36 (d, 1H), 2.98 (q, 2H), 1.08 (t, 3H); MS *m*/*z :* 459[M+H]

### Example 10. 5-ethyl-N-(3-fluoro-4-((1-isopropyl-2-oxo-2,3-dihydro-1H-imidazo[4,5-b]pyridine-7-yl)oxy)phenyl)-1-phenyl-1H-pyrazole-4-carboxamide

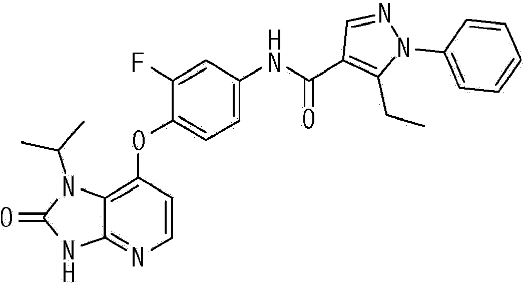

A title compound (26 mg, yield: 53%) was obtained in a manner similar to that of Example 7 by using 5-ethyl-1-phenyl-1H-pyrazole-4-carboxylic acid instead of 1-phenyl-5-(trifluoromethyl)-1H-pyrazole-4-carboxylic acid according to Example 7.
¹H NMR (500 MHz, DMSO-d₆) δ 11.68 (brs, 1H), 10.15 (s, 1H), 8.34 (s, 1H), 8.01 (d, 1H), 7.82 (d, 1H), 7.60 (m, 3H), 7.51 (m, 3H), 7.45 (t, 1H), 6.41 (d, 1H), 4.90 (m, 1H), 2.99 (q, 2H), 1.09 (t, 3H); MS *m*/*z* : 501[M+H]

### Example 11. N-(3-fluoro-4-((2-oxo-1-(tetrahydro-2H-pyran-4-yl)-2,3-dihydro-1H-imidazo [4,5-b] pyridine-7-yl)oxy)phenyl)-1-phenyl-5-(trifluoromethyl)-1H-imidazole-4-carboxamide

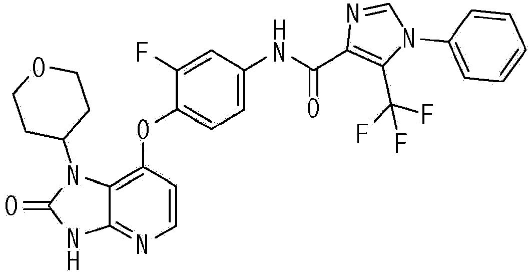

A title compound (1.4 mg, yield: 12%) was obtained in a manner similar to that of Example 1 by using 7-(4-amino-2-fluorophenoxy)-1-(tetrahydro-2H-pyran-4-yl)-1,3-dihydro-2H-imidazo[4,5-b]pyridine-2-one instead of 7-(4-amino-2-fluorophenoxy)-1-H-1,3-dihydro-2H-imidazo[4,5-b]pyridine-2-one according to Example 1.

MS *m*/*z* : 583[M+H].

### Example 12. N-(3-fluoro-4-((2-oxo-2,3-dihydro-1H-imidazo[4,5-b]pyridine-7-yl)oxy)phenyl)-3,5-dimethyl-1-phenyl-1H-pyrazole-4-carboxamide

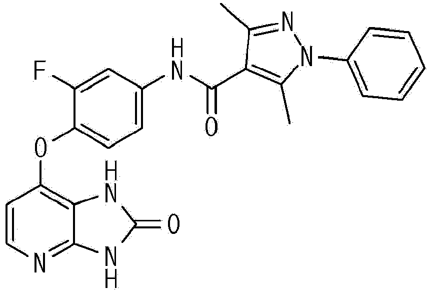

A title compound (25 mg, yield: 48%) was obtained in a manner similar to that of Example 1 by using 3,5-dimethyl-1-phenyl-1H-pyrazole-4-carboxylic acid instead of 1-phenyl-5-(trifluoromethyl)-1H-imidazole-4-carboxylic acid according to Example 1.
¹H NMR (500 MHz, DMSO-d₆) δ 11.41 (brs, 1H), 11.27 (brs, 1H), 10.14(s, 1H), 7.93 (d, 1H), 7.77 (d, 1H), 7.57 (m, 2H), 7.52 (m, 4H), 7.34 (t, 1H), 6.35 (d, 1H), 2.43 (s, 3H), 2.38 (s, 3H); MS *m*/*z :* 459[M+H]

### Example 13. N-(3-fluoro-4-((2-oxo-2,3-dihydro-1H-imidazo[4,5-b]pyridine-7-yl)oxy)phenyl)-3,5-dimethyl-1-phenyl-1H-pyrazole-4-carboxamide

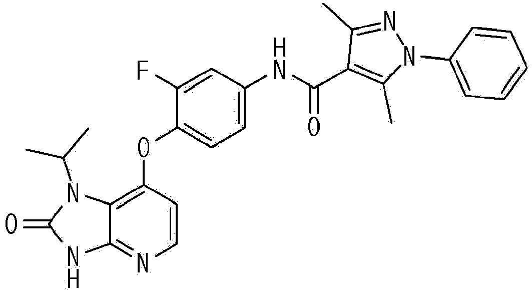

A title compound (9 mg, yield: 22%) was obtained in a manner similar to that of Example 7 by using 3,5-dimethyl-1-phenyl-1H-pyrazole-4-carboxylic acid instead of 1-phenyl-5-(trifluoromethyl)-1H-pyrazole-4-carboxylic acid according to Example 7.
¹H NMR (500 MHz, MeOH-d₄) δ 7.92 (d, 1H), 7.86 (d, 1H), 7.61 (m, 2H), 7.49 (m, 4H), 7.37 (t, 1H), 6.49 (d, 1H), 5.08 (m, 1H), 2.48 (s, 3H), 2.46 (s, 3H), 1.62 (d, 6H); MS *m*/*z* : 501[M+H]

### Example 14. N-(3-fluoro-4-((1-isopropyl-2-oxo-2,3-dihydro-1H-imidazo [4,5-b] pyridine-7-yl)oxy)phenyl)-5-m ethyl-1-phenyl-1H-pyrazole-4-carboxamide

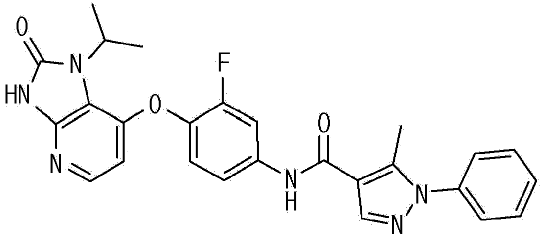

A title compound (15 mg, yield: 23%) was obtained in a manner similar to that of Example 7 by using 5-methyl-1-phenyl-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 10.15 (s, 1H), 8.34 (s, 1H), 7.98 (dd, 1s), 7.87 (d, 1H), 7.61-7.51 (m, 6H), 7.43 (t, 1H), 6.40 (d, 1H), 4.88(m, 1H), 2.57 (s, 3H), 1.50 (1s, 3H), 1.49 (1s, 3H); MS *m*/*z* : 487[M+H]

### Example 15. N-(3-fluoro-4-((1-isopropyl-2-oxo-2,3-dihydro-1H-imidazo[4,5-b]pyridine-7-yl)oxy)phenyl)-2-phenylthiazole-5-carboxamide

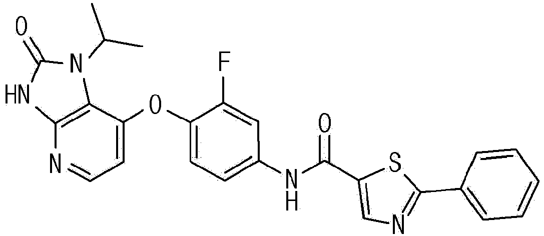

A title compound (20 mg, yield: 31%) was obtained in a manner similar to that of Example 7 by using 2-phenylthiazole-5-carboxylic acid instead of 1-phenyl-5-(trifluoromethyl)-1H-pyrazole-4-carboxylic acid according to Example 7.

¹H NMR (500 MHz, DMSO-d₆) δ 11.70 (s, 1H), 10.77 (s, 1H), 8.71 (s, 1H), 8.06-8.05 (m, 2H), 7.95 (dd, 1H), 7.82 (d, 1H), 7.62-7.56 (m, 4H), 7.47 (t, 1H), 6.41 (d, 1H), 4.87 (m, 1H), 1.49 (s, 3H), 1.48 (s, 3H); MS *m*/*z* : 490[M+H].

### Example 16. N-(3-fluoro-4-((1-isopropyl-2-oxo-2,3-dihydro-1H-imidazo[4,5-b]pyridine-7-yl)oxy)phenyl)-5-phenylthiazole-2-carboxamide

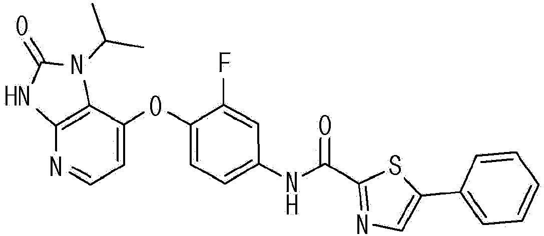

A title compound (10 mg, yield: 18%) was obtained in a manner similar to that of Example 7 by using 5-phenylthiazole-5-carboxylic acid instead of 1-phenyl-5-(trifluoromethyl)-1H-pyrazole-4-carboxylic acid according to Example 7.

¹H NMR (500 MHz, DMSO-d₆) δ 11.69 (s, 1H), 11.17 (s, 1H), 8.56 (s, 1H), 8.06 (dd, 1H), 7.86-7.80 (m, 4H), 7.54-7.44 (m, 4H), 6.41 (d, 1H), 4.87 (m, 1H), 1.49 (s, 3H), 1.47 (s, 3H); MS *m*/*z :* 490[M+H].

### Example 17. N-(3-fluoro-4-((1-isopropyl-2-oxo-2,3-dihydro-1H-imidazo [4,5-b] pyridine-7-yl)oxy)phenyl)-1-(p-tolyl)-5-(trifluoromethyl)-1H-pyrazole-4-carboxamide

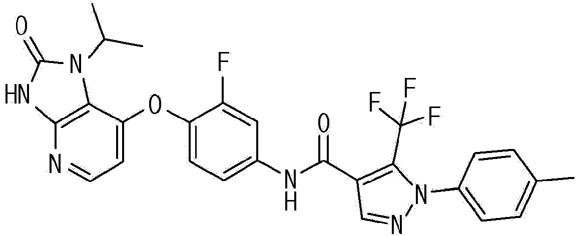

A title compound (15 mg, yield: 20%) was obtained in a manner similar to that of Example 7 by using 1-(p-tolyl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (400 MHz, DMSO-d₆) δ 11.65 (s, 1H), 10.80 (s, 1H), 8.27 (s, 1H), 7.88 (dd, 1H), 7.76 (d, 1H), 7.52 (d, 1H), 7.46-7.35 (m, 5H), 6.36 (d, 1H), 4.83 (m, 1H), 2.31 (s, 3H), 1.45 (s, 3H), 1.43 (s, 3H); MS *m*/*z :* 555[M+H].

### Example 18. N-(3-fluoro-4-((1-isopropyl-2-oxo-2,3-dihydro-1H-imidazo[4,5-b]pyridine-7-yl)oxy)phenyl)-1-(2-methoxyphenyl)-5-(trifluoromethyl)-1H-pyrazole-4-carboxamide

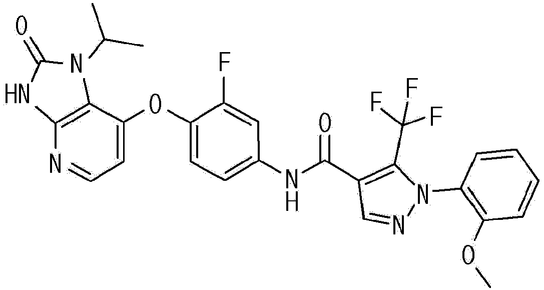

A title compound (30 mg, yield: 40%) was obtained in a manner similar to that of Example 7 by using 1-(2-methoxyphenyl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (400 MHz, DMSO-d₆) δ 11.55 (brs, 1H), 10.73 (s, 1H), 8.30 (s, 1H), 7.89 (dd, 1H), 7.76 (d, 1H), 7.58-7.53 (m, 2H), 7.43-7.38 (m, 2H), 7.25 (d, 1H), 7.10 (t, 1H), 6.36 (d, 1H), 4.83 (m, 1H), 3.75 (s, 3H), 1.44 (s, 3H), 1.43 (s, 3H); MS *m*/*z* : 571[M+H].

### Example 19. N-(3-fluoro-4-((1-isopropyl-2-oxo-2,3-dihydro-1H-imidazo[4,5-b]pyridine-7-yl)oxy)phenyl)-1,5-diphenyl-1H-pyrazole-4-carboxamide

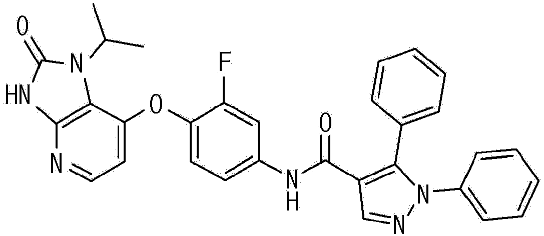

A title compound (14 mg, yield: 19%) was obtained in a manner similar to that of Example 7 by using 1,5-diphenyl-1H-pyrazole-4-carboxylic acid instead of 1-phenyl-5-(trifluoromethyl)-1H-pyrazole-4-carboxylic acid according to Example 7.

¹H NMR (400MHz, DMSO-d₆) δ 10.23 (s, 1H), 8.35 (s, 1H), 7.83 (dd, 1H), 7.74 (d, 1H), 7.46 (d, 1H), 7.36-7.26 (m, 9H), 7.19 (dd, 2H), 6.31 (d, 1H), 4.81(m, 1H), 1.43 (s, 3H), 1.42 (s, 3H); MS *m*/*z* : 549[M+H].

### Example 20. N-(3-fluoro-4-((1-isopropyl-2-oxo-2,3-dihydro-1H-imidazo[4,5-b]pyridine-7-yl)oxy)phenyl)-1-(tetrahydro-2H-pyran-3-yl)-5-(trifluoromethyl)-1H-pyrazole-4-carboxamide

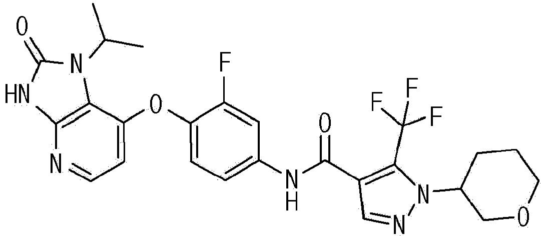

A title compound (24 mg, yield: 33%) was obtained in a manner similar to that of Example 7 by using 1-(tetrahydro-2H-pyran-3-yl)-5-(trifluoromethyl)-1H-pyrazole-4-carboxylic acid instead of 1-phenyl-5-(trifluoromethyl)-1H-pyrazole-4-carboxylic acid according to Example 7.

MS *m*/*z :* 549[M+H].

### Example 21. N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide

A title compound (35 mg, yield: 64%) was obtained in a manner similar to that of Example 1 by using 7-(4-amino-2-fluorophenoxy)-1-cyclopentyl-1,3-dihydro-2H-imidazo[4,5-b]pyridine-2-one obtained in Preparation Example 4 instead of 7-(4-amino-2-fluorophenoxy)-1-H-1,3-dihydro-2H-imidazo[4,5-b]pyridine-2-one according to Example 1.

¹H NMR (400 MHz, DMSO-d₆) δ 11.68 (s, 1H), 10.74 (s, 1H), 8.31 (s, 1H), 7.99 (dd, 1H), 7.77 (d, 1H), 7.71 (d, 1H), 7.59-7.54 (m, 5H), 7.39 (t, 1H), 6.36 (d, 1H), 4.94 (m, 1H), 2.08-1.99 (m, 2H), 1.94-1.87 (m, 2H), 1.71-1.69 (m, 2H), 1.59-1.54 (m, 2H); MS *m*/*z :* 567[M+H].

### Example 22. N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide

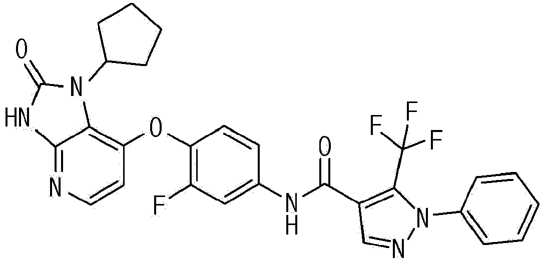

A title compound (20 mg, yield: 29%) was obtained in a manner similar to that of Example 21 by using 1-phenyl-5-(trifluoromethyl)-1H-pyrazole-4-carboxylic acid instead of 1-phenyl-5-(trifluoromethyl)-1H-imidazole-4-carboxylic acid according to Example 21.

¹H NMR (400 MHz, DMSO-d₆) δ 11.72 (s, 1H), 10.85 (s, 1H), 8.34 (s, 1H), 7.92 (dd, 1H), 7.82 (d, 1H), 7.64-7.63 (m, 3H), 7.57-7.55 (m, 3H), 7.46 (t, 1H), 6.41 (d, 1H), 2.09-2.05 (m, 2H), 1.98-1.97 (m, 2H), 1.76-1.75 (m, 2H), 1.61-1.60 (m, 2H); MS *m*/*z* : 567[M+H].

### Example 23. N-(4-((1-cyclopentyl-2-oxo-2,3-dihydro-1H-imidazo[4,5-b]pyridine-7-yl)oxy)-3-fluorophenyl)-5-ethyl-1-phenyl-1H-pyrazole-4-carboxamide

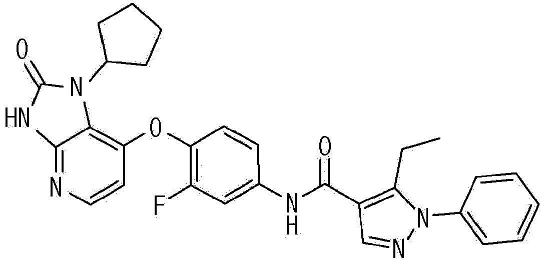

A title compound (22 mg, yield: 36%) was obtained in a manner similar to that of Example 21 by using 5-ethyl-1-phenyl-1H-pyrazole-4-carboxylic acid instead of 1-phenyl-5-(trifluoromethyl)-1H-imidazole-4-carboxylic acid according to Example 21.

¹H NMR (400 MHz, DMSO-d₆) δ 11.68 (s, 1H), 10.11 (s, 1H), 8.29 (s, 1H), 7.94 (dd, 1H), 7.78 (d, 1H), 7.58-7.46 (m, 6H), 7.38 (t, 1H), 6.36 (d, 1H), 4.95 (m, 1H), 2.92 (q, 2H), 2.05-2.00 (m, 2H), 1.94-1.90 (m, 2H), 1.71-1.69 (m, 2H), 1.56-1.55 (m, 2H), 1.03 (t, 3H); MS *m*/*z :* 527[M+H].

### Example 24. N-(3-fluoro-4-((1-isopropyl-2-oxo-2,3-dihydro-1H-imidazo[4,5-b]pyridine-7-yl)oxy)phenyl)-2-(trifluoromethyl)benzamide

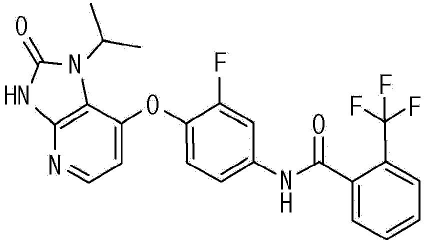

A title compound (32 mg, yield: 51%) was obtained in a manner similar to that of Example 7 by using 2-(trifluoromethyl)benzoic acid instead of 1-phenyl-5-(trifluoromethyl)-1H-pyrazole-4-carboxylic acid according to Example 7.

¹H NMR (500 MHz, DMSO-d₆) δ 11.68 (s, 1H), 10.90 (s, 1H), 7.91-7.74 (m, 6H), 7.53 (d, 1H), 7.44 (t, 1H), 6.42 (d, 1H), 4.87 (m, 1H), 1.49 (s, 3H), 1.47 (s, 3H); MS *m*/*z* : 475[M+H].

### Example 25. 4-bromo-N-(3-fluoro-4-((1-isopropyl-2-oxo-2,3-dihydro-1H-imidazo[4,5-b]pyridine-7-yl)oxy)phenyl)-2-(trifluoromethyl)benzamide

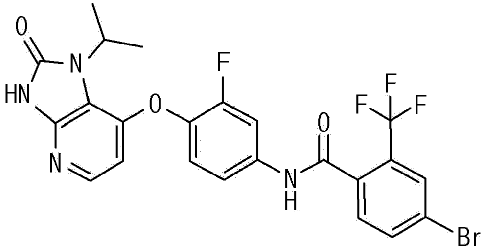

A title compound (27 mg, yield: 37%) was obtained in a manner similar to that of Example 7 by using 4-bromo-2-(trifluoromethyl)benzoic acid instead of 1-phenyl-5-(trifluoromethyl)-1H-pyrazole-4-carboxylic acid according to Example 7.

^{,1}H NMR (500 MHz, DMSO-d₆) δ 11.69 (s, 1H), 10.94 (s, 1H), 8.11 (s, 1H), 8.07 (d, 1H), 7.87 (dd, 1H), 7.81 (d, 1H), 7.73 (d, 1H), 7.50 (d, 1H), 7.45 (t, 1H), 6.41 (d, 1H), 4.87 (m, 1H), 1.48 (s, 3H), 1.47 (s, 3H); MS *m*/*z* : 553[M], 555[M+2].

### Example 26. N-(3-fluoro-4-((1-isopropyl-2-oxo-2,3-dihydro-1H-imidazo[4,5-b]pyridine-7-yl)oxy)phenyl)-1-(naphthalene-1-yl)-5-(trifluoromethyl)-1H-pyrazole-4-carboxamide

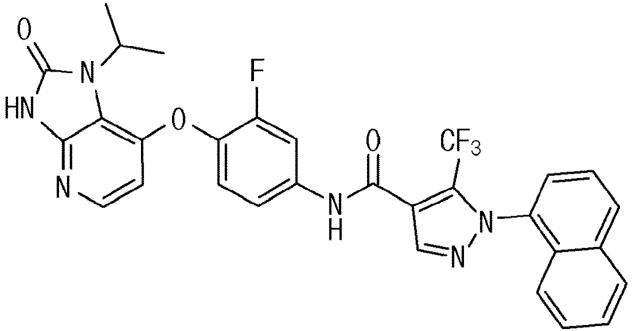

A title compound (45 mg, yield: 58%) was obtained in a manner similar to that of Example 7 by using 1-(naphthalen-1-yl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.69 (s, 1H), 10.90(s, 1H), 8.49 (s, 1H), 8.25 (d, 1H), 8.15 (d, 1H), 7.95 (dd, 1H), 7.81 (d, 1H), 7.78 (d, 1H), 7.77-7.59 (m, 4H), 7.48 (t, 1H), 7.13 (d, 1H), 6.43 (d, 1H), 4.88 (m, 1H), 1.50 (s, 3H), 1.48 (s, 1H); MS *m*/*z :* 591 [M+H].

### Example 27. 3-bromo-N-(3-fluoro-4-((1-isopropyl-2-oxo-2,3-dihydro-1H-imidazo[4,5-b]pyridine-7-yl)oxy)phenyl)-2-(trifluoromethyl)benzamide

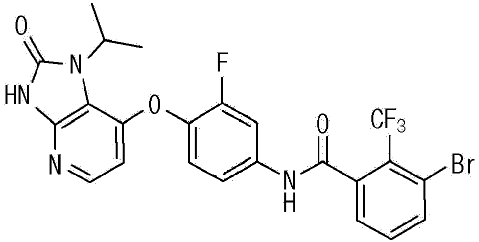

A title compound (100 mg, yield: 27%) was obtained in a manner similar to that of Example 7 by using 3-bromo-2-(trifluoromethyl)benzoic acid instead of 1-phenyl-5-(trifluoromethyl)-1H-pyrazole-4-carboxylic acid according to Example 7.

¹H NMR (500 MHz, DMSO-d₆) δ 11.69 (s, 1H), 10.96 (s, 1H), 8.05 (d, 1H), 7.86-7.73 (m, 2H), 7.70 (m, 2H), 7.49-7.43 (m, 2H), 6.41 (d, 1H), 4.87 (m, 1H), 1.48 (s, 3H), 1.47 (s, 3H); MS *m*/*z* : 553[M], 555[M+2].

### Example 28. 1-cyclohexyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide

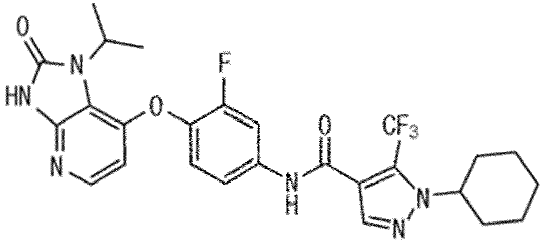

A title compound (24 mg, yield: 33%) was obtained in a manner similar to that of Example 7 by using 1-cyclohexyl-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.70 (s, 1H), 10.76 (s, 1H), 8.07 (s, 1H), 7.88 (d, 1H), 7.80 (d, 1H), 7.51 (d, 1H), 7.43 (t, 1H), 6.39 (d, 1H), 1.94-1.85 (m, 6H), 1.70-1.68 (m, 1H), 1.48 (s, 3H), 1.47 (s, 3H), 1.43-1.40 (m, 1H), 1.28-1.20 (m, 2H); MS *m*/*z* : 547[M+H].

### Example 29. N-(3-fluoro-4-((1-isopropyl-2-oxo-2,3-dihydro-1H-imidazo[4,5-b]pyridine-7-yl)oxy)phenyl)-1-(tetrahydro-2H-pyran-4-yl)-5-(trifluoromethyl)-1H-pyrazole-4-carboxamide

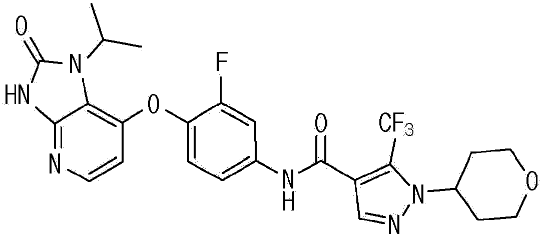

A title compound (41 mg, yield: 57%) was obtained in a manner similar to that of Example 7 by using 1-(tetrahydro-2H-pyran-4-yl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.70 (s, 1H), 10.78 (s, 1H), 8.11 (s, 1H), 7.88 (dd, 1H), 7.80 (d, 1H), 7.51 (d, 1H), 7.43 (t, 1H), 6.39 (d, 1H), 4.78 (m, 1H), 4.62 (m, 1H), 3.99 (dd, 2H), 3.54 (t, 2H), 2.15 (m, 2H), 1.90 (dd, 2H), 1.48 (s, 3H), 1.47 (s, 3H); MS *m*/*z* : 549[M+H].

### Example 30. 1-(tert-butyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide

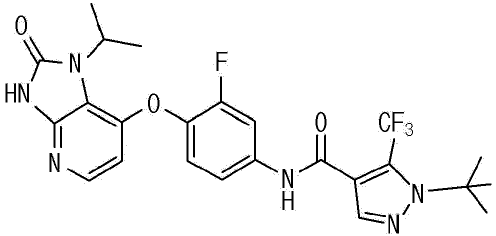

A title compound (30 mg, yield: 44%) was obtained in a manner similar to that of Example 7 by using 1-(tert-butyl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.70 (s, 1H), 10.82(s, 1H), 7.96 (s, 1H), 7.88 (dd, 1H), 7.50 (d, 1H), 7.43 (t, 1H), 6.39 (d, 1H), 4.86 (m, 1H), 1.67 (s, 9H), 1.48 (s, 3H), 1.47 (s, 3H); MS *m*/*z* : 520[M+H].

### Example 31. 1-cycloheptyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide

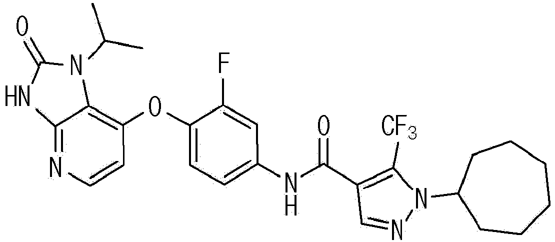

A title compound (28 mg, yield: 38%) was obtained in a manner similar to that of Example 7 by using 1-cycloheptyl-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.69 (s, 1H), 10.76(s, 1H), 8.06 (s, 1H), 7.88 (d, 1H), 7.80 (d, 1H), 7.51 (d, 1H), 7.43 (t, 1H), 6.39 (d, 1H), 4.86 (m, 1H), 4.52 (m, 1H), 2.10-1.99 (m, 4H), 1.81 (m, 1H), 1.62-1.53 (m, 6H), 1.48 (s, 3H), 1.47 (s, 3H); MS *m*/*z* : 561[M+H].

### Example 32. N-(3-fluoro-4-((1-isopropyl-2-oxo-2,3-dihydro-1H-imidazo[4,5-b]pyridine-7-yl)oxy)phenyl)-2-(1-phenyl-5-(trifluoromethyl)-1H-pyrazol-4-yl)acetamide

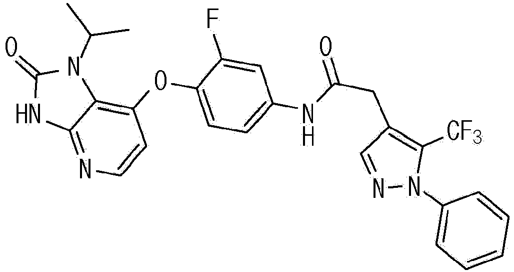

A title compound (16 mg, yield: 22%) was obtained in a manner similar to that of Example 7 by using 2-(1-phenyl-5-(trifluoromethyl)-1H-pyrazol-4-yl)acetic acid instead of 1-phenyl-5-(trifluoromethyl)-1H-pyrazole-4-carboxylic acid according to Example 7.

¹H NMR (500 MHz, DMSO-d₆) δ 10.90 (s, 1H), 8.90 (d, 1H), 8.39 (s, 1H), 7.90 (d, 1H), 7.77 (d, 1H), 7.68 (d, 1H), 7.53 (d, 1H), 7.38 (t, 1H), 6.43 (s, 2H), 5.96 (d, 1H), 2.61 (s, 3H), 2.55 (s, 3H); MS *m*/*z* : 555[M+H].

### Example 33. N-(3-fluoro-4-((1-isopropyl-2-oxo-2,3-dihydro-1H-imidazo[4,5-b]pyridine-7-yl)oxy)phenyl)-1-(1-methylpiperidine-3-yl)-5-(trifluoromethyl)-1H-pyrazole-4-carboxamide

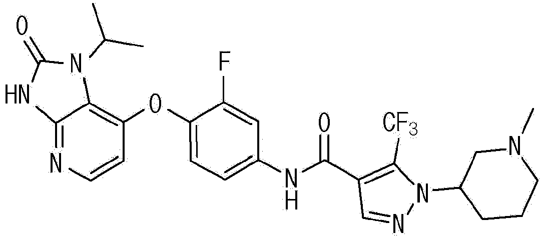

A title compound (2 mg, yield: 4%) was obtained in a manner similar to that of Example 7 by using 1-(1-methylpiperidine-3-yl)-5-(trifluoromethyl)-1H-pyrazole-4-carboxylic acid instead of 1-phenyl-5-(trifluoromethyl)-1H-pyrazole-4-carboxylic acid according to Example 7.

MS *m*/*z* : 562[M+H].

### Example 34. N-(3-fluoro-4-((1-isopropyl-2-oxo-2,3-dihydro-1H-imidazo[4,5-b]pyridine-7-yl)oxy)phenyl)-1-(2-fluorophenyl)-5-(trifluoromethyl)-1H-pyrazole-4-carboxamide

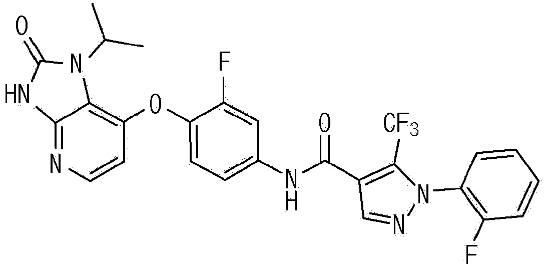

A title compound (25 mg, yield: 45%) was obtained in a manner similar to that of Example 7 by using the compound 1-(2-fluorophenyl)-5-(trifluoromethyl)-1H-pyrazole-4-carboxylic acid obtained in Preparation Example 6 instead of 1-phenyl-5-(trifluoromethyl)-1H-pyrazole-4-carboxylic acid according to Example 7.

¹H NMR (500 MHz, DMSO-d₆) δ 11.70 (s, 1H), 10.87 (s, 1H), 8.44 (s, 1H), 7.93 (dd, 1H), 7.81 (d, 1H), 7.73 (m, 2H), 7.58 (m, 2H), 7.76 (m, 2H), 6.41 (d, 1H), 4.87 (m, 1H), 1.49 (s, 3H), 1.48 (s, 3H); MS *m*/*z :* 559[M+H].

### Example 35. 1-(2chlorophenyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide

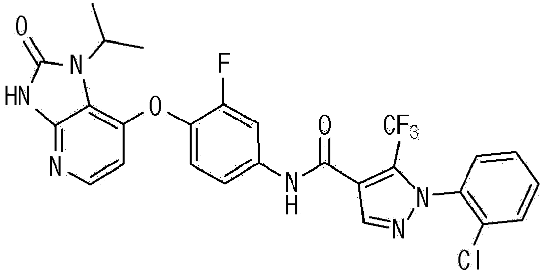

A title compound (12 mg, yield: 21%) was obtained in a manner similar to that of Example 7 by using 1-(2-chlorophenyl)-5-(trifluoromethyl)-1H-pyrazole-4-carboxylic acid instead of 1-phenyl-5-(trifluoromethyl)-1H-pyrazole-4-carboxylic acid according to Example 7.

¹H NMR (500 MHz, DMSO-d₆) δ 11.69 (s, 1H), 10.84 (s, 1H), 8.45 (s, 1H), 7.94 (d, 1H), 7.80 (m, 2H), 7.71 (m, 2H), 7.60 (m, 2H), 7.46 (t, 1H), 6.41 (d, 1H), 4.87 (m, 1H), 1.49 (s, 3H), 1.48 (s, 3H); MS *m*/*z* : 575[M+H].

### Example 36. 1-(2,6-difluorophenyl)-N-(3-fluoro-4-((1-isopropyl-2-oxo-2,3-dihydro-1H-imidazo[4,5- b]pyridine-7-yl)oxy)phenyl)-5-(trifluoromethyl)-1H-pyrazole-4-carboxamide

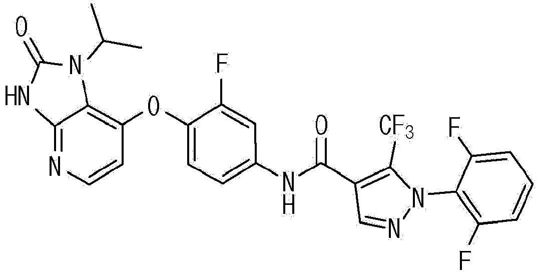

A title compound (22 mg, yield: 39%) was obtained in a manner similar to that of Example 7 by using 1-(2,6-difluorophenyl)-5-(trifluoromethyl)-1H-pyrazole-4-carboxylic acid instead of 1-phenyl-5-(trifluoromethyl)-1H-pyrazole-4-carboxylic acid according to Example 7.
1H NMR (500 MHz, DMSO-d6) δ 11.68 (s, 1H), 10.93 (s, 1H), 8.52 (s, 1H), 7.92 (d, 1H), 7.87-7.81 (m, 2H), 7.57-7.51 (m, 3H), 7.46 (t, 1H), 6.41 (d, 1H), 4.87 (m, 1H), 1.49 (s, 3H), 1.48 (s, 3H); MS m/z : 577[M+H]

### Example 37. N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-pyrazole-4-carboxamide

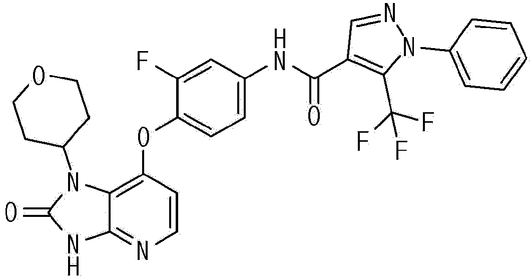

A title compound (30 mg, yield: 59%) was obtained in a manner similar to that of Example 7 by using 7-(4-amino-2-fluorophenoxy)-1-(tetrahydro-2H-pyran-4-yl)-1,3-dihydro-2H-imidazo[4,5-b]pyridine- 2-one instead of 7-(4-amino-2-fluorophenoxy)-1-isopropyl-1,3-dihydro-2H-imidazo[4,5-b]pyridine-2-one according to Example 7.
1H NMR (500 MHz, DMSO-d6) δ 11.78 (brs, 1H), 10.86 (s, 1H), 8.35 (s, 1H), 7.94 (d, 1H), 7.83 (d, 1H), 7.63 (m, 3H), 7.57 (m, 3H), 7.47 (m, 1H), 6.43 (d, 1H), 4.71 (m, 1H), 3.97 (m, 2H), 3.43 (m, 2H), 2.43 (m, 2H), 1.73 (m, 2H); MS m/z : 583[M+H]

### Example 38. N-(3-fluoro-4-((2-oxo-1-(tetrahydrofuran-3-yl)-2,3-dihydro-1H-imidazo[4,5-b]pyridine-7-yl)oxy)phenyl)-1-phenyl-5-(trifluoromethyl)-1H-pyrazole-4-carboxamide

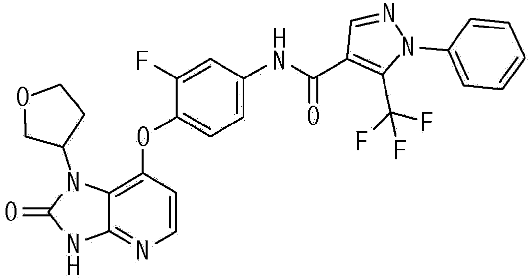

A title compound (25 mg, yield: 49%) was obtained in a manner similar to that of Example 7 by using 7-(4-amino-2-fluorophenoxy)-1-(tetrahydrofuran-3-yl)-1,3-dihydro-2H-imidazo[4,5-b]pyridine-2-one instead of 7-(4-amino-2-fluorophenoxy)-1-isopropyl-1,3-dihydro-2H-imidazo[4,5-b]pyridine-2-one according to Example 7.
1H NMR (500 MHz, DMSO-d6) δ 11.81 (s, 1H), 10.85 (s, 1H), 8.35 (s, 1H), 7.94 (d, 1H), 7.84 (m, 1H), 7.63 (m, 3H), 7.58 (m, 3H), 7.47 (m, 1H), 6.43 (d, 1H), 5.29 (m, 1H), 4.03 (m, 1H), 3.93 (m, 3H), 2.43 (m, 1H), 2.29 (m, 1H); MS m/z : 569[M+H]

### Example 39. N-(3-fluoro-4-((1-(1-methylpiperidine-4-yl)-2-oxo-2,3-dihydro-1H-imidazo[4,5-b]pyridine-7-yl)oxy)phenyl)-1-phenyl-5-(trifluoromethyl)-1H-pyrazole-4-carboxamide

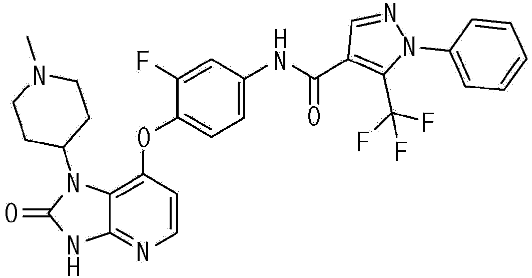

A title compound (0.7 mg, yield: 4%) was obtained in a manner similar to that of Example 7 by using 7-(4-amino-2-fluorophenoxy)-1-(1-methylpiperidine-4-yl)-1,3-dihydro-2H-imidazo[4,5-b]pyridine-2-one instead of 7-(4-amino-2-fluorophenoxy)-1-isopropyl-1,3-dihydro-2H-imidazo[4,5-b]pyridine-2-one according to Example 7.
MS m/z : 596[M+H]

### Example 40. N-(3-fluoro-4-((1-isopropyl-2-oxo-2,3-dihydro-1H-imidazo[4,5-b]pyridine-7-yl)oxy)phenyl)-1-phenyl-1H-pyrazole-3-carboxamide

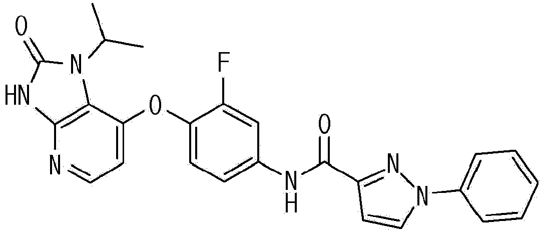

A title compound (27 mg, yield: 48%) was obtained in a manner similar to that of Example 7 by using 1-phenyl-1H-pyrazole-3-carboxylic acid instead of 1-phenyl-5-(trifluoromethyl)-1H-pyrazole-4-carboxylic acid according to Example 7.

1H NMR (500 MHz, DMSO-d6) δ 11.67 (s, 1H), 10.47 (s, 1H), 8.68 (s, 1H), 8.12 (d, 1H), 8.07 (m, 2H), 7.81 (m, 2H), 7.60 (m, 2H), 7.43 (m, 2H), 7.08 (s, 1H), 6.41 (d, 1H), 4.86 (m, 1H), 1.49 (d, 6H).

### Example 41. N-(3-fluoro-4-((1-isopropyl-2-oxo-2,3-dihydro-1H-imidazo[4,5-b]pyridine-7-yl)oxy)phenyl)-1-phenyl-1H-1,2,3-triazole-4-carboxamide

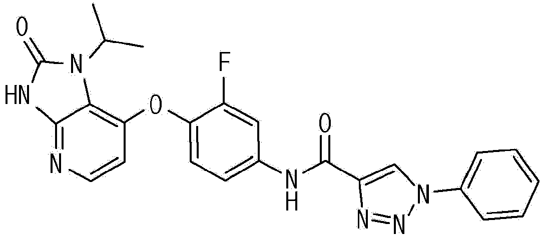

A title compound (27 mg, yield: 48%) was obtained in a manner similar to that of Example 7 by using 1-phenyl-1H-1,2,3-triazole-4-carboxylic acid instead of 1-phenyl-5-(trifluoromethyl)-1H-pyrazole-4-carboxylic acid according to Example 7.

1H NMR (500 MHz, DMSO-d6) δ 11.69 (s, 1H), 10.97 (s, 1H), 9.50 (s, 1H), 8.09 (m, 3H), 7.81 (s, 2H), 7.67 (m, 2H), 7.58 (m, 1H), 7.47 (m, 1H), 6.41 (d, 1H), 4.86 (m, 1H), 1.49 (d, 6H).

### Example 42. N-(3-fluoro-4-((1-isopropyl-2-oxo-2,3-dihydro-1H-imidazo[4,5-b]pyridine-7-yl)oxy)phenyl)-5-methyl-1-phenyl-1H-1,2,3-triazole-4-carboxamide

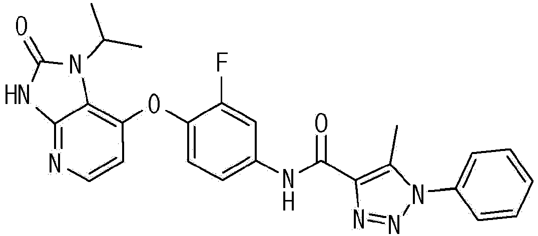

A title compound (35 mg, yield: 61%) was obtained in a manner similar to that of Example 7 by using 5-methyl-1-phenyl-1H-1,2,3-triazole-4-carboxylic acid instead of 1-phenyl-5-(trifluoromethyl)-1H-pyrazole-4-carboxylic acid according to Example 7.

1H NMR (500 MHz, DMSO-d6) δ 11.68 (s, 1H), 10.90 (s, 1H), 8.10 (d, 1H), 7.84 (t, 2H), 7.68 (m, 5H), 7.45 (t, 1H), 6.41 (d, 1H), 4.86 (m, 1H), 2.60 (s, 3H), 1.49 (d, 6H).

### Example 43. N-(3-fluoro-4-((1-isopropyl-2-oxo-2,3-dihydro-1H-imidazo[4,5-b]pyridine-7-yl)oxy)phenyl)-1-(2-fluorophenyl)-1H-1,2,3-triazole-4-carboxamide

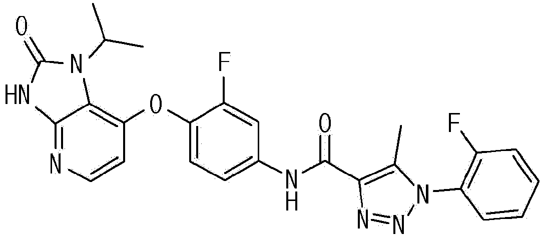

A title compound (38 mg, yield: 64%) was obtained in a manner similar to that of Example 7 by using 1-(2-fluorophenyl)-5-methyl-1H-1,2,3-triazole-4-carboxylic acid instead of 1-phenyl-5-(trifluoromethyl)-1H-pyrazole-4-carboxylic acid according to Example 7.

1H NMR (500 MHz, DMSO-d6) δ 11.69 (s, 1H), 10.94 (s, 1H), 8.09 (d, 1H), 7.84 (m, 4H), 7.65 (m, 1H), 7.54 (m, 1H), 7.46 (m, 1H), 6.41 (d, 1H), 4.86 (m, 1H), 2.50 (s, 3H), 1.49 (d, 6H).

### Experimental Example 1. Evaluation of inhibitory activity against RON and MET

### [1-1] Evaluation of inhibitory activity against RON

In order to measure the inhibitory activity of each of the compounds of Examples against RON, the IC50 of each of the compounds was measured using a Time-Resolved Fluorescence Energy Transfer Chemistry (TR-FRET).

Specifically, the compound was prepared to a concentration of 1 mM with 100% DMSO and was diluted 10 times in a stepwise manner through 3-fold dilution. 2 ul of the compound diluted in a stepwise manner was added to a 96-well-plate containing 48ul of a 1x kinase reaction buffer (50 mM HEPES (pH 7.4), 0.01% Tween-20, 5 mM DTT, 0.5 mM Na₃VO₄, 2 mM EGTA, 10 mM MgCl₂).

The RON protein was diluted to a concentration of 49.3 nM with an RON storage buffer (50 mM Tris-HCl (pH 7.5), 150 mM NaCl, 0.5 mM EDTA, 0.02% Triton X-100), and then diluted again to a concentration of 0.4 nM with a 1x kinase reaction buffer. As a substrate cocktail, a RON-specific substrate mixture (40uM ULight-labeled Poly GT, 100 nM ATP) at a concentration 2 times the final reaction concentration.

Thereafter, a 384-well-plate was prepared, and 2.5 µl of each of the diluted compounds of Examples was added to a corresponding well of experimental group wells, and 2.5 µl of a 4% DMSO solution was dispensed into high control wells and low control wells.

Thereafter, 2.5 µL of 0.4 nM RON was dispensed into the high control wells and the experimental group wells, and 2.5 µL of a 1x kinase reaction buffer was added to the low control wells, and the RON and the reaction buffer were centrifuged at room temperature for 40 seconds at 1000 RPM, and then incubated at room temperature for 20 to 30 minutes. Then, 5uL of a substrate cocktail (2x) was dispensed into all wells, centrifuged at room temperature for 40 seconds at 1000 RPM, and then incubated at room temperature for 60 minutes. Thereafter, 5 uL of 30 mM EDTA was added to all wells to terminate the reaction, and then incubated again at room temperature for 5 minutes. Thereafter, 5 uL of a 4x phosphotyrosine antibody (Perkin Elmer) containing europium was added to all wells, and then incubated at room temperature for 60 minutes. After the incubation, the 384-well-plate was read with a Vison plate reader. At this time, the excitation wavelength was 340 nm, and the emission wavelength was 620 nm and 665 nm. The IC50 value of the compound of each Example was derived using the GraphPd Prism7 program.

### [1-2] Evaluation of inhibitory activity against MET

In addition, in order to measure the enzyme activity inhibition for cMET, the evaluation was performed in the same manner as the enzyme activity inhibition measurement experiment for the RON.

Specifically, the compound was prepared to a concentration of 1 mM with 100% DMSO and was diluted 10 times in a stepwise manner through 3-fold dilution. 2 ul of the compound diluted in a stepwise manner was added to a 96-well-plate containing 48ul of a 1x kinase reaction buffer (50 mM HEPES (pH 7.4), 0.05% BSA, 0.005 % Tween-20, 1 mM DTT, 0.5 mM MnCl₂, 20 mM MgCl₂).

The cMET protein was diluted to a concentration of 263 nM with a cMET storage buffer (50 mM Tris-HCl (pH 7.5), 150 mM NaCl, 0.05% Brij35, 1 mM DTT, 10% Glycerol), and then diluted again to a concentration of 2 nM with a 1x kinase reaction buffer. As a substrate cocktail, a MET-specific substrate mixture (5 uM TK peptide, 10 mM ATP) at a concentration 2 times the final reaction concentration.

Thereafter, a 384-well-plate was prepared, and 2.5 µl of each of the diluted compounds of Examples was added to a corresponding well of experimental group wells, and 2.5 µl of a 4% DMSO solution was dispensed into high control wells and low control wells.

Thereafter, 2.5 µL of 2 nM cMET was dispensed into the high control wells and the experimental group wells, and 2.5 µL of a 1x kinase reaction buffer was added to the low control wells, and the RON and the reaction buffer were centrifuged at room temperature for 40 seconds at 1000 RPM, and then incubated at room temperature for 20 to 30 minutes. Then, 5uL of a substrate cocktail (2x) was dispensed into all wells, centrifuged at room temperature for 40 seconds at 1000 RPM, and then incubated at room temperature for 60 minutes. Thereafter, 5 uL of 90 mM EDTA was added to all wells to terminate the reaction, and then incubated again at room temperature for 5 minutes. Thereafter, 5 uL of a 4x phosphotyrosine antibody (Perkin Elmer) containing europium was added to all wells, and then incubated at room temperature for 60 minutes. After the incubation, the 384-well-plate was read with a Vison plate reader. At this time, the excitation wavelength was 340 nm, and the emission wavelength was 620 nm and 665 nm. The IC50 value of the compound of each Example was derived using the GraphPd Prism7 program.

The results of evaluating the inhibitory activity of the compounds of Examples are shown in Table 2 below.
(A: <50 nM, B: 50 to 500 nM, C: 500 to 5000 nM, D: >5,000 nM)

**[Table 2]**

| | RON (IC₅₀, nM) | MET (IC₅₀, nM) |
|---|---|---|
| Example 1 | A | D |
| Example 2 | A | D |
| Example 3 | A | D |
| Example 4 | A | D |
| Example 5 | A | D |
| Example 6 | A | D |
| Example 7 | A | C |
| Example 8 | A | D |
| Example 9 | B | D |
| Example 10 | A | C |
| Example 11 | A | D |
| Example 12 | B | D |
| Example 13 | A | B |
| Example 14 | A | C |
| Example 15 | B | D |
| Example 16 | B | D |
| Example 17 | B | D |
| Example 18 | B | C |
| Example 19 | B | B |
| Example 20 | A | C |
| Example 21 | A | C |
| Example 22 | A | C |
| Example 23 | B | C |
| Example 24 | C | C |
| Example 25 | B | D |
| Example 26 | B | C |
| Example 27 | C | D |
| Example 28 | A | C |
| Example 29 | B | B |
| Example 30 | B | C |
| Example 31 | A | C |
| Example 32 | B | C |
| Example 33 | B | C |
| Example 34 | A | C |
| Example 35 | A | C |
| Example 36 | A | C |
| Example 37 | B | C |
| Example 38 | A | C |
| Example 39 | A | D |
| Example 40 | C | C |
| Example 41 | C | D |
| Example 42 | B | D |
| Example 43 | A | D |
| A: <50 nM, B: 50 to 500 nM, C: 500 to 5000 nM, D: >5,000 nM | | |

## Claims

1. A urea derivative compound of Formula 1 below or a pharmaceutically acceptable salt thereof: 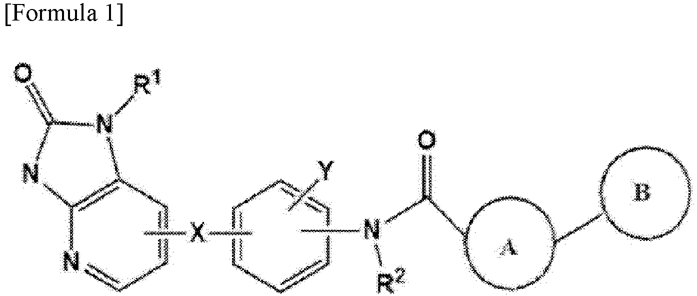 wherein in the Formula 1 above,
X above is oxygen,
Y above is hydrogen, halogen, C₁-C₆ alkyl, or C₃-C₆ cycloalkyl,
R¹ above is hydrogen, C₁-C₆ alkyl, C₃-C₁₂ cycloalkyl, or C₂-C₁₁ heterocycloalkyl,
R² above is hydrogen or C₁-C₆ alkyl,
A above is -Ar or -(CH₂)n-Ar, wherein Ar is substituted or unsubstituted C₆-C₁₀ aryl, or substituted or unsubstituted 5-membered heteroaryl including 1 to 4 heterocyclic atoms selected from the group consisting of nitrogen, sulfur, and oxygen, wherein a substituent is one or more selected from halogen, amine, C₁-C₆ alkyl, C₃-C₁₂ cycloalkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR³R⁴, and n above is an integer of 1 to 3,
R³ and R⁴ above are each independently hydrogen or C₁-C₆ alkyl,
B above is hydrogen, C₁-C₆ alkyl, substituted C₁-C₆ alkyl, C₃-C₁₂ cycloalkyl, substituted C₃-C₁₂ cycloalkyl, C₃-C₁₁ heterocycloalkyl, substituted C₃-C₁₁ heterocycloalkyl, C₆-C₂₀ aryl, substituted C₆-C₂₀ aryl, wherein a substituent is halogen, C₁-C₆ alkyl, C₃-C₇ cycloalkyl, halogen-substituted C1-C6 alkyl, C₁-C₆ alkoxy, C₃-C₁₁ heterocycloalkyl, C₃-C₁₁ heterocycloalkenyl, or C₆-C₁₀ aryl,
wherein the halogens are each independently selected from the group consisting of F, Cl, Br, and I.

2. The compound of claim 1 or a pharmaceutically acceptable salt thereof, wherein the compound is represented by Formula 2 below: 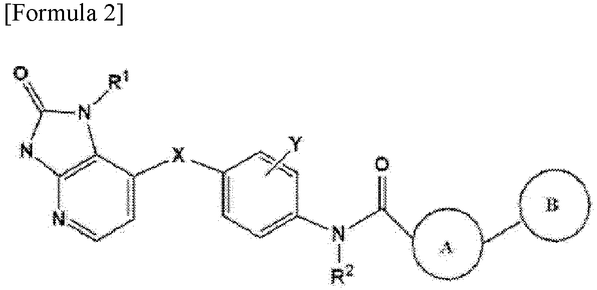 wherein the substituent is the same as defined in the Formula 1.

3. The compound of claim 1 or a pharmaceutically acceptable salt thereof, wherein A above is a substituted or unsubstituted 5-membered heteroaryl.

4. The compound of claim 1 or a pharmaceutically acceptable salt thereof, wherein B above is hydrogen, C₁-C₆ alkyl, substituted C₁-C₆ alkyl, C₃-C₇ cycloalkyl, phenyl, substituted phenyl, benzyl, or naphthalene tetrahydropyran, piperidine or substituted piperidine, wherein a substituent is halogen, C₁-C₃ alkyl, halogen-substituted C₁-C₃ alkyl, C₃-C₅ cycloalkyl, or C₁-C₃ alkoxy.

5. The compound of claim 1 or a pharmaceutically acceptable salt thereof, wherein Y above is fluoro.

6. The compound of claim 1 or a pharmaceutically acceptable salt thereof, wherein R¹ above is hydrogen, C₁-C₆ alkyl, C₃-C₆ cycloalkyl, or C₄-C₆ heterocycloalkyl.

7. The compound of claim 1 or a pharmaceutically acceptable salt thereof, wherein R² above is hydrogen.

8. The compound of claim 1 or a pharmaceutically acceptable salt thereof, wherein the compound is selected from the group consisting of
N-(3-fluoro-4-((2-oxo-2,3-dihydro-1H-imidazo[4,5-b]pyridine-7-yl)oxy)phenyl)-1-phenyl-5-(trifluoromethyl)-1H-imidazole-4-carboxamide,
N-(3-fluoro-4-((2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide,
N-(4-((1-ethyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide,
N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-imidazole-4-carboxamide,
N-(3-fluoro-4-((1-methyl-2-oxo-2,3-dihydro-1H-imidazo[4,5-b]pyridine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-1-phenyl-5-(trifluoromethyl)-1H-imidazole-4-carboxamide,
5-ethyl-N-(3-fluoro-4-((2-oxo-2,3-dihydro-1H-imidazo[4,5-b]pyridine-7-yl)oxy)phenyl)-1-phenyl-1H-pyrazole-4-carboxamide,
5-ethyl-N-(3-fluoro-4-((1-isopropyl-2-oxo-2,3-dihydro-1H-imidazo[4,5-b]pyridine-7-yl)oxy)phenyl)-1-phenyl-1H-pyrazole-4-carboxamide,
N-(3-fluoro-4-((2-oxo-1-(tetrahydro-2H-pyran-4-yl)-2,3-dihydro-1H-imidazo[4,5-b]pyridine-7-yl)oxy)phenyl)-1-phenyl-5-(trifluoromethyl)-1H-imidazole-4-carboxamide,
N-(3-fluoro-4-((2-oxo-2,3-dihydro-1H-imidazo[4,5-b]pyridine-7-yl)oxy)phenyl)-3,5-dimethyl-1-phenyl-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-3, 5-dimethyl-1-phenyl-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-5-methyl-1-phenyl-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-2-phenylthiazole-5-carboxamide,
N-(3-fluoro-4-((1-isopropyl-2-oxo-2,3-dihydro-1H-imidazo[4, 5-b]pyridine-7-yl)oxy)phenyl)-5-phenylthiazole-2-carboxamide,
N-(3-fluoro-4-((1-isopropyl-2-oxo-2,3-dihydro-1H-imidazo[4, 5-b]pyridine-7-yl)oxy)phenyl)-1-(p-tolyl)-5-(trifluoromethyl)-1H-pyrazole-4-carboxamide
N-(3-fluoro-4-((1-isopropyl-2-oxo-2,3-dihydro-1H-imidazo[4, 5-b]pyridine-7-yl)oxy)phenyl)-1-(2-methoxyphenyl)-5-(trifluoromethyl)-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-1, 5-diphenyl-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-1-(tetrahydro-2H-pyran-3-yl)-5-(trifluoromethyl)-1H-pyrazole-4-carboxamide,
N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-imidazole-4-carboxamide,
N-(4-((1-cyclopentyl-2-oxo-2,3-dihydro-1H-imidazo[4,5-b]pyridine-7-yl)oxy)-3-fluorophenyl)-1-phenyl-5-(trifluoromethyl)-1H-pyrazole-4-carboxamide,
N-(4-((1-cyclopentyl-2-oxo-2,3-dihydro-1H-imidazo[4,5-b]pyridine-7-yl)oxy)-3-fluorophenyl)-5-ethyl-1-phenyl-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-2-(trifluoromethyl)benzamide,
4-bromo-N-(3-fluoro-4-((1-isopropyl-2-oxo-2,3-dihydro-1H-imidazo[4,5 - b]pyridine-7-yl)oxy)phenyl)-2-(trifluoromethyl)benzamide,
N-(3-fluoro-4-((1-isopropyl-2-oxo-2,3-dihydro-1H-imidazo[4, 5-b]pyridine-7-yl)oxy)phenyl)-1-(naphthalene-1-yl)-5-(trifluoromethyl)-1H-pyrazole-4-carboxamide,
3 -bromo-N-(3-fluoro-4-((1-isopropyl-2-oxo-2,3-dihydro-1H-imidazo[4,5 - b]pyridine-7-yl)oxy)phenyl)-2-(trifluoromethyl)benzamide,
1-cyclohexyl-N-(3-fluoro-4-((1-isopropyl-2-oxo-2,3-dihydro-1H-imidazo[4,5-b]pyridine-7-yl) Oxy)phenyl)-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-(tetrahydro-2H-pyran-4-yl)-5-(trifluoromethyl)-1H-pyrazole-4-carboxamide,
1-(tert-butyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
1-cycloheptyl-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-2-(1-phenyl-5-(trifluoromethyl)-1H-pyrazol-4-yl)acetamide,
N-(3-fluoro-4-((1-isopropyl-2-oxo-2,3-dihydro-1H-imidazo[4, 5-b]pyridine-7-yl)oxy)phenyl)-1-(1-methylpiperidine-3-yl)-5-(trifluoromethyl)-1H-pyrazole-4-carboxamide,
N-(3-fluoro-4-((1-isopropyl-2-oxo-2,3-dihydro-1H-imidazo[4, 5-b]pyridine-7-yl)oxy)phenyl)-1-(2fluorophenyl)-5-(trifluoromethyl)-1H-pyrazole-4-carboxamide,
1-(2chlorophenyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
1-(2,6-difluorophenyl)-N-(3-fluoro-4-((1-isopropyl-2-oxo-2,3-dihydro-1H-imidazo[4,5-b]pyridine-7-yl)oxy)phenyl)-5-(trifluoromethyl)-1H-pyrazole-4-carboxamide,
N-(3-fluoro-4-((2-oxo-1-(tetrahydro-2H-pyran-4-yl)-2,3-dihydro-1H-imidazo[4,5-b]pyri dine-7-yl)oxy)phenyl)-1-phenyl-5-(trifluoromethyl)-1H-pyrazole-4-carboxamide,
N-(3-fluoro-4-((2-oxo-1-(tetrahydrofuran-3-yl)-2,3-dihydro-1H-imidazo[4,5-b]pyri dine-7-yl)oxy)phenyl)-1-phenyl-5-(trifluoromethyl)-1H-pyrazole-4-carboxamide,
N-(3-fluoro-4-((1-(1-methylpiperidine-4-yl)-2-oxo-2,3-dihydro-1H-imidazo[4,5-b]pyri dine-7-yl)oxy)phenyl)-1-phenyl-5-(trifluoromethyl)-1H-pyrazole-4-carboxamide,
N-(3-fluoro-4-((1-isopropyl-2-oxo-2,3-dihydro-1H-imidazo[4,5-b]pyridine-7-yl)oxy)phenyl)-1-phenyl-1H-pyrazole-3-carboxamide,
N-(3-fluoro-4-((1-isopropyl-2-oxo-2,3-dihydro-1H-imidazo[4,5-b]pyridine-7-yl)oxy)phenyl)-1-phenyl-1H-1,2,3-triazole-4-carboxamide,
N-(3-fluoro-4-((1-isopropyl-2-oxo-2,3-dihydro-1H-imidazo[4,5-b]pyridine-7-yl)oxy)phenyl)-5-methyl-1-phenyl-1H-1,2,3-triazole-4-carboxamide,
and
N-(3-fluoro-4-((1-isopropyl-2-oxo-2,3-dihydro-1H-imidazo[4, 5-b]pyridine-7-yl)oxy)phenyl)-1-(2-fluorophenyl)-1H-1,2,3-triazole-4-carboxamide.

9. A pharmaceutical composition comprising the compound according to any one of claims 1 to 8 or a pharmaceutically acceptable salt thereof, and a pharmaceutically acceptable carrier.
